# EUROPEAN PATENT APPLICATION

(11) **EP 4 502 607 A2**
(43) Date of publication of application: **05.02.2025**
(21) Application number: 24205039.1
(22) Date of filing: 09.02.2015
(51) Int. Cl.: G01N 33/68

(54) **PROTEOMIC SAMPLE PREPARATION USING PARAMAGNETIC BEADS**

(30) Priority: 07.02.2014 EP 14154326; 19.02.2014 EP 14155853
(62) Divisional of application: 15703093.3
(71) Applicant: European Molecular Biology Laboratory, 69117 Heidelberg (DE)
(72) Inventor: KRIJGSVELD, Jeroen, 74925 Epfenbach (DE); STEINMETZ, Lars, 69115 Heidelberg (DE); HUGHES, Christopher, Vancouver, BC V5Z 1L3 (CA)
(74) Representative: Dentons Patent Solutions Rechtsanwaltsgesellschaft mbH

(57) **Abstract**

The present invention relates to a method of reversibly binding polypeptides to a solid phase comprising a hydrophilic surface, preferably for the use in mass spectrometry based proteomics, Kits providing reagents for the method of the invention and uses of said kits.

## Description

The present invention relates to a method of reversibly binding polypeptides to a solid phase comprising a hydrophilic surface, preferably for the use in mass spectrometry based proteomics, Kits providing reagents for the method of the invention and uses of said kits.

### Background of the Invention

Current generation of proteomics strives to obtain complete characterization of the proteome for a range of cellular systems, types and subtypes (Lamond, A.L. *et al.,* 2012). This includes, but is not limited to examination, synthesis, degradation, abundance, and post-translational modifications such as phosphorylation for all proteoforms (Smith, L. M. *et al.,* 2013). Rapid advancements in hardware and software have seen mass spectrometry (MS) develop into one of the primary detection methods utilized in proteomics (Yates, J.R. *et al.,* 2009). Diversity and complexity in cellular proteomes has driven the development of a broad range of protocols to support their analysis by MS. These methods are optimized to improve the depth of proteome coverage through generation of conditions that are favourable for proteolytic digestion and sample recovery. Whether experimenting *in vivo, in vitro* or on purified extracts one commonality between all support protocols is careful selection of solutions and enrichment methods during sample preparation to ensure compatibility with downstream workflows and detection platforms. In spite of the fact that most detergents (e.g. SDS) are incompatible with enzymatic digestions and downstream MS-analysis, they are often utilized to achieve protein solubilization. Ultrafiltration (Filter-assisted-Sample-Preparation, FASP) (Wisniewski, J.R. *et al.,* 2009) or bead-based protocols (Hengel, S.M. *et al.,* 2012; Bereman, M.S. *et al.,* 2011) are efficient for removal of detergents, but have minimal flexibility and require significant sample handling (filtration, centrifugation, concentration), resulting in sample losses and reduced overall efficiency. Therefore, the development of an adaptable platform that enhances these collective properties would be a considerable advancement in the field of proteomics. The rapid expansion of next-generation sequencing has prompted the development of protocols amenable to high-throughput genome library preparation, greatly facilitated by the application of paramagnetic beads in manual and roboticized platforms (Hawkins, T.L. *et al.,* 1994; DeAngelis, M.M. *et al.,* 1995; Wilkening, S. *et al.,* 2013). The flexibility and ease of handling offered by paramagnetic beads promotes the generation of simplified protocols that gamer widespread utility. The use of paramagnetic beads in proteomics is far less widespread, with the primary applications focused on targeted approaches, such as affinity-based pull-downs and immobilization strategies for recombinant proteins or proteolytic enzymes. Recently, methods have been developed based on functionalized nanodiamond particles, facilitating depletion of contaminating substances (e.g. detergents) and enhancement of compartment-specific proteomics analysis (Chen, W.-H. et al., 2006). However, limitations related to the conditions required for binding (SDS < 0.5%) limit the universality of this approach. Building on technology developments pioneered by solid-phase reversible immobilization (SPRI) nucleic acid clean-up and nanodiamond technologies, we have developed a universal and streamlined platform built on paramagnetic beads, herein referred to as SP3 (single-pot solid-phase-enhanced sample preparation). SP3 is based on the unbiased immobilization of proteins and peptides on carboxylate functionalized beads coated with a hydrophilic surface. The beads utilized in SP3 are commercially available and do not require pretreatment under oxidizing conditions, such as with nanodiamond particles. Immobilization is promoted through trapping proteins and peptides in an aqueous solvation layer on the hydrophilic bead surface through increasing the concentration of organic additive in solution. Once on the bead surface, proteins and peptides can be processed and rinsed in a flexible format to remove contaminants, such as detergents. Using SP3, we successfully demonstrate that proteins can be immobilized on paramagnetic beads in an unbiased fashion in the presence of a variety of compounds, such as detergents (up to 10% SDS), reducing agents (DDT and BME, > 1M), urea (8M), and a variety of other salts and buffers. The tolerance against high detergent concentrations in the binding buffer, e.g. up to 10% SDS represents a significant advantage to the nanodiamond method. Further, the method allows binding of proteins in a non-selective mode, contrary to commonly used methods wherein only proteins exhibiting certain properties (e.g. membrane- 25 binding proteins) absorb. Contaminating substances can be removed by rinsing with a combination of organic solvents. The purified proteins can be eluted from the beads through the addition of an aqueous solution, and directly enzymatically digested. Resulting peptides can be used in downstream HPLC-based fractionation methods. Alternatively, the peptides can be re-immobilized on the paramagnetic beads for sample clean-up prior to MS-analysis, thus 30 eliminating common sample handling steps, such as rota-evaporation. Moreover, immobilized peptides can be fractionated off the beads and directly injected to an MS, thus enabling a true-single-tube proteomics workflow. Aside from reducing sample losses by minimizing sample handling and transfer, the established workflow is also rapid, requiring only 15 minutes each, for protein and peptide clean-up and a further 15 minutes for fractionation. Capitalizing on the sensitivity afforded with this single-tube workflow, we can effectively perform in-depth proteome characterization of large sample amounts, but also confidently detect >3000 unique proteins by MS starting with just 1000 intact HeLa cells, This depth-of-coverage represents a 10-fold improvement of currently published results and simultaneously underlines the utility of this workflow when it comes to applications where limited amounts of sample are available. Thus, this workflow represents a significant advance in conventional proteomics analysis, and as well as in ultra-sensitive applications using rare or limiting cell types.

### Summary of the Invention

In a first aspect the present invention provides a method of reversibly binding polypeptides to a solid phase comprising a hydrophilic surface, comprising the step of:
a) contacting said solid phase, a solution containing said polypeptides and a dehydration solution and/or a precipitation solution.

In a second aspect the present invention provides a kit comprising:
(i) a solid phase comprising a hydrophilic surface , and
(ii) a dehydration solution and/or a precipitation solution.

In a third aspect the invention provides the use of a kit of the second aspect of the invention.

Furthermore, the present invention provides the following items:
1. A method of reversibly binding polypeptides to a solid phase comprising a hydrophilic surface, comprising the step:
   a) of contacting said solid phase, a solution containing said polypeptides; and a dehydration solution and/or a precipitation solution.
2. The method of item 1, wherein the hydrophilic surface is a polymer with hydrophilic properties, preferably polyacrylamide, polyacrylic acid, polyacrylimide, polyelectrolytes, polyethylenimin, polyethylenglycol, polyethylenoxid, polyvinylalcohol, polyvinylpyrrolidon polystyrene sulfonic acid, copolymers of styrene and maleic acid, vinyl methyl ether malic acid copolymer, and polyvinylsulfonic acid or comprises a hydrophilic compound selected from the group consisting of aminoethylmethacrylate, carbohydrate, cucurbit[n]uril hydrate, dimethylaminomethyl methacrylate, fumaric acid, maleic acid, methacrylic acid, isopropylacrylamid, itaconic acid, N-vinyl carbazole, 4-pentenoic acid, polyalkylene glycol diamine, pyrrol, t-butylaminoethyl methacrylate, undecylenic acid, vinyl acetic acid, and vinylpyrdidine.
3. The method of items 1 or 2, wherein the hydrophilic surface further comprises a negatively charged moiety selected from the group comprising a carboxylic acid moiety, silica moiety, a sulphate moiety, a phosphate moiety, nitrite moiety, nitrate moiety.
4. The method of any of items 1 to 3, wherein the solid phase comprises microbeads or microparticles.
5. The method of item 1, wherein the dehydration solution comprises a polar organic solvent and/or a hygroscopic polymer,
   preferably the polar aprotic organic solvent is selected from the group consisting of acetonitrile, acetone, alcohol, dichloromethane, dimethylformamide (DMF), dimethylsulfoxide (DMSO), ethylacetate, hexamethylphosphoramide (HPMA), and tetrahydrofuran (THF), preferably the hygroscopic polymer is selected from the group consisting of polyethylene glycol, dextranes, alginates, cellulose, polyacrylic acid, tannic acid, and glycogen.
6. The method of any of items 1 to 5, wherein the dehydration solution and/or precipitation solution is added in an amount resulting in the reversible binding of at least 90% of the polypeptides in said solution.
7. The method of any of items 1 to 6, wherein the solution containing said polypeptides is selected from a whole cell extract, a whole cell extract digest, proteins derived from tissue, recombinant purified proteins, purified proteins, a protein digest, a purified protein digest, and a peptide library.
8. The method of any of items 1 to 7, wherein the solid phase comprising reversibly bound polypeptides is separated from the solution.
9. The method of item 8, wherein the solid phase is washed with a washing solution comprising a polar organic solvent or a salt or chaotrope comprising at least one ion that increases hydrophobic interaction of polypeptides, preferably in a concentration range from 1M - 8 M.
10. The method of any of items 1 to 9, comprising the further step of adding a protease.
11. The method of any of items 1 to 10, wherein the polypeptides are released from the solid phase with an aqueous elution solution.
12. The method of any of items 10 or 11, comprising the further step of submitting the aqueous solution to mass spectrometry.
13. Kit comprising:
   (i) a solid phase comprising a hydrophilic surface , and
   (ii) a dehydration solution and/or a precipitation solution.
14. Kit of claim 13, further comprising:
   (i) reagents for labeling polypeptides, preferably including stable isotopic labeling components,
   (ii) reagents for eluting polypeptides, preferably aqueous buffers
   (iii) reagents for digestion of polypeptides, preferably proteases and detergents;
   (iv) suitable reagents for fractionation of polypeptides;
   (v) reagents for determining protein concentration;
   (vi) suitable reagents for glycopeptide or phosphopeptide enrichment, and/or
   (vii) suitable reagents for cell lysis.
15. Use of a solid phase comprising a hydrophilic surface or a kit of any of items 13 to 14, for the analysis, identification, characterisation, quantification, purification, concentration and/or separation of polypeptides.

### List of Figures

In the following, the content of the figures comprised in this specification is described. In this context please also refer to the detailed description of the invention above and/or below.

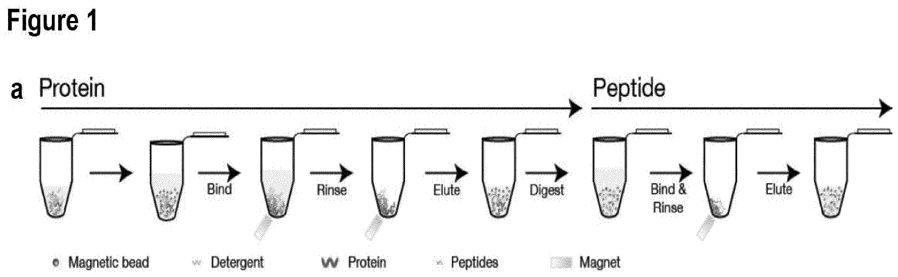

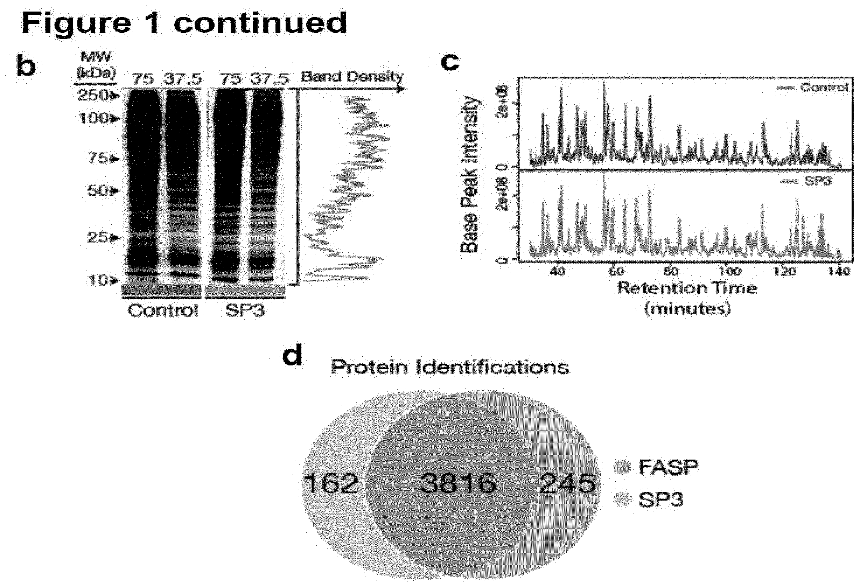

### Figure 1: Protein and peptide enrichment with SP3.

Protein and peptide mixtures can be efficiently bound, rinsed, and eluted using paramagnetic beads in the SP3 protocol. (a) Workflow of SP3 sample preparation. Protein mixtures are bound through mixing with paramagnetic beads and adding a specified amount of formic acid and acetonitrile (50% v/v final concentration). After incubation, proteins bound on beads can be rinsed with organic solutions to remove contaminants such as detergents. Purified proteins can be eluted through the addition of aqueous solution to the bead mixture. Peptide mixtures can be bound in the same manner as proteins, using an adjustment to 95% (v/v) of acetonitrile. All steps in the SP3 protocol can be carried out in a single tube with an individual set of beads. (b) SDS-PAGE gel displaying recovery of a protein mixture left untreated (Control), or treated with SP3. Numbers at the top of the lanes depict the total amount of starting material in micrograms. Plot on the right illustrates the band densitometry overlap between the 37.5µg Control and SP3 lanes. (c) Base peak chromatograms from analysis of a peptide mixture directly injected (Control) or treated with SP3 prior to injection to the MS. (d) Unique protein identification overlap between in-depth proteomic datasets obtained from samples treated with SP3 or FASP protocols.

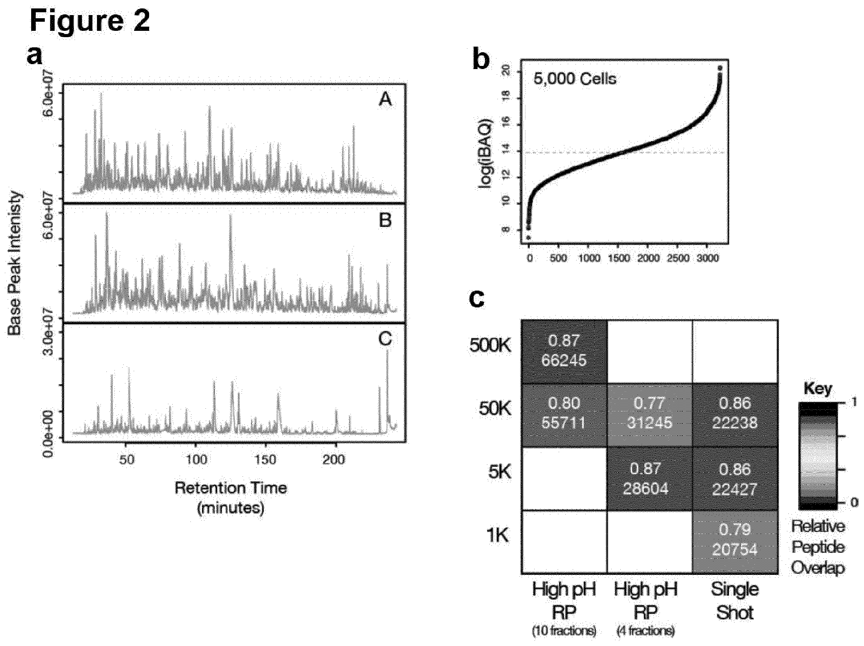

### Figure 2: Ultra-sensitive proteomics with SP3.

SP3 is compatible with the analysis of rare or limiting cell types. Differing numbers of HeLa cells (500000, 50000, 5000, and 1000) were lysed with SDS, treated with SP3 at the protein and peptide level, and either fractionated using high-pH reversed phase on an HPLC, high-pH reversed phase on a StageTip, or as a single shot injection. (a) Base peak chromatograms for single shot injections at the different cell amounts, where (A) is 50,000, (B) is 5,000, and (C) is 1,000. (b) Protein abundance estimates based on iBAQ values. With 5,000 cells and a single shot injection, proteins across a wide range of protein abundance are sampled in the analysis. (c) Unique peptide recovery and reproducibility for different fractionation types and cell numbers. Intensity of the color indicates the higher percentage overlap between replicates based on unique peptide sequences. Numbers indicated in each box represent the percentage overlap between replicates (above) and the total number of unique peptides identified from combined replicates (below).

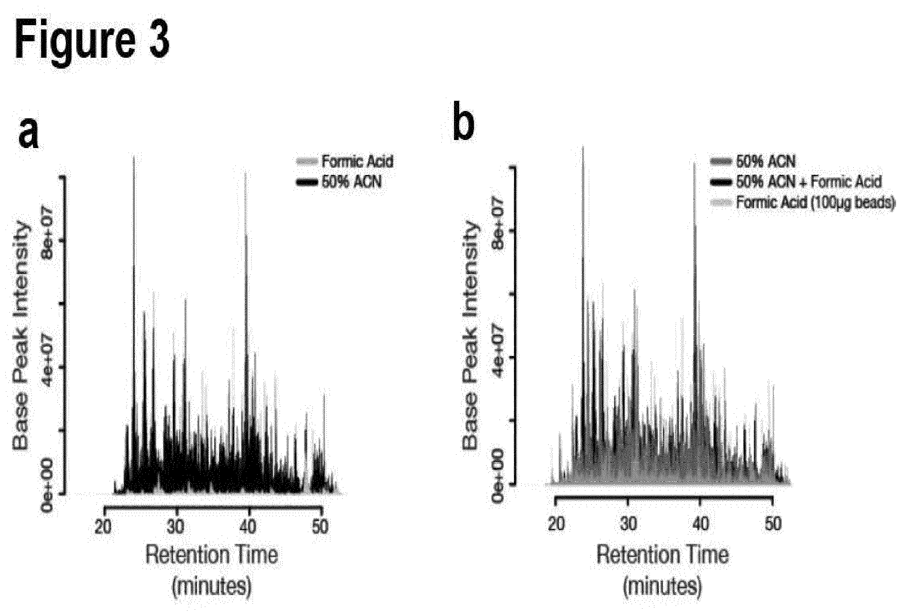

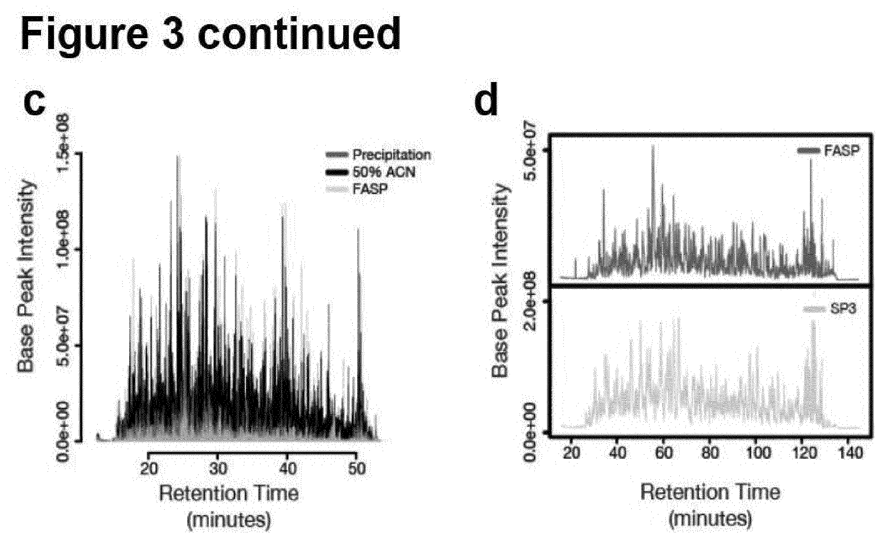

### Figure 3: Binding of proteins with SP3 protocol.

Proteins can be efficiently captured and rinsed using carboxylate modified paramagnetic beads in the SP3 protocol. Equivalent aliquots of a yeast whole-cell lysate (--10ug of total protein) in SDS-containing buffer were treated with acidic (nanodiamond), acetonitrile (SP3), and salt (SPRI) conditions. Recovered proteins were digested and the resulting peptides run on an HCT-ion trap MS. (a) MS base peak chromatograms of a digested yeast lysates. Protein-bead mixtures were acidified with formic acid or volumetrically adjusted to a concentration of 50% acetonitrile (ACN). Bound proteins were equivalently rinsed, recovered, digested, and injected to the MS. (b) MS base peak chromatograms of yeast lysates volumetrically adjusted to 50% ACN with or without formic acid, or treated with formic acid in the presence of a high concentration of beads. (c) MS base peak chromatograms of yeast lysates treated with high salt (SPRI), ACN (SP3), or with FASP. Equivalent amounts of recovered peptides based on the amount of starting material were injected. (d) Extended chromatographic runs of yeast lysates treated with the optimized SP3 protein protocol (50% ACN with formic acid) and FASP.

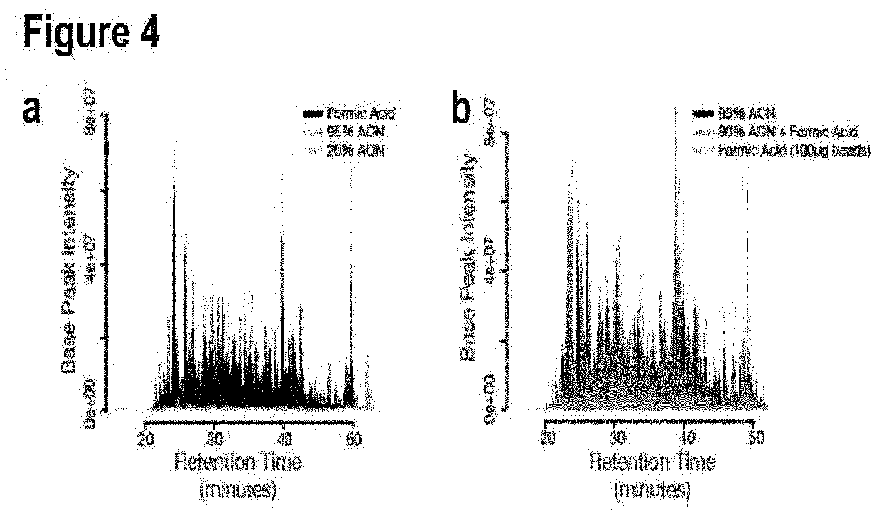

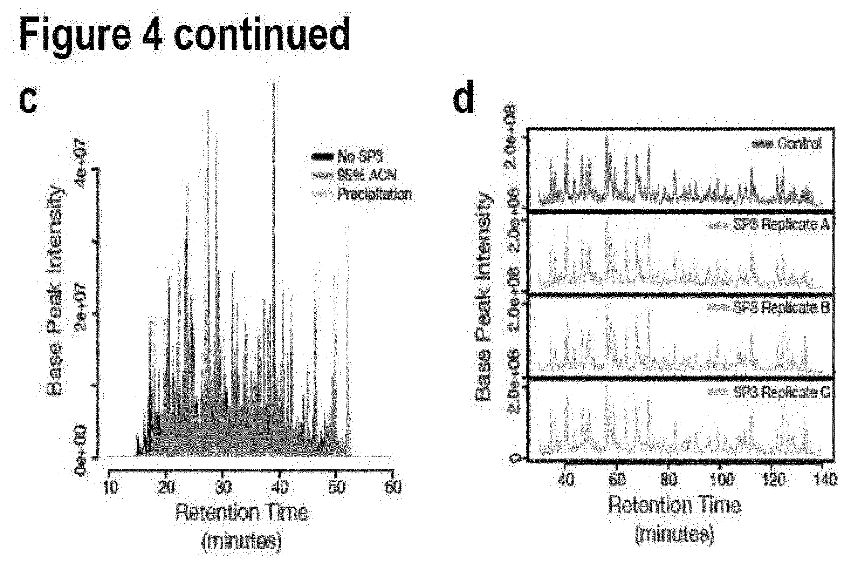

### Figure 4: Binding of peptides with SP3 protocol.

Peptides can be efficiently captured and rinsed using carboxylate modified paramagnetic beads in the SP3 protocol. Equivalent aliquots of a yeast whole-cell lysate digested with trypsin and rLysC were treated with acidic (nanodiamond), acetonitrile (SP3), and salt (SPRI) conditions. Recovered peptides were run on an HCT-ion trap MS. (a) MS base peak chromatograms of a digested yeast lysates. Peptide-bead mixtures were acidified with formic acid or volumetrically adjusted to a concentration of 20% or 95% acetonitrile (ACN). Bound peptides were equivalently rinsed, recovered, and injected to the MS. (b) MS base peak chromatograms of peptides volumetrically adjusted to 95% ACN, 90% ACN with formic acid, or treated with formic acid in the presence of a high concentration of beads. (c) MS base peak chromatograms of yeast lysates treated with high salt (SPRI), ACN (SP3), or directly injected without treatment (No SP3). (d) Extended chromatographic runs of peptide replicates treated with the optimized SP3 peptide protocol (95% ACN) or StageTips (Control).

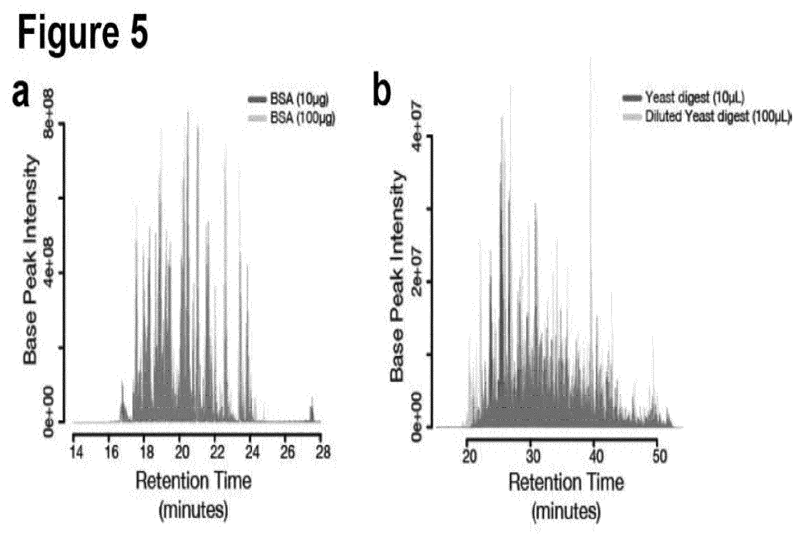

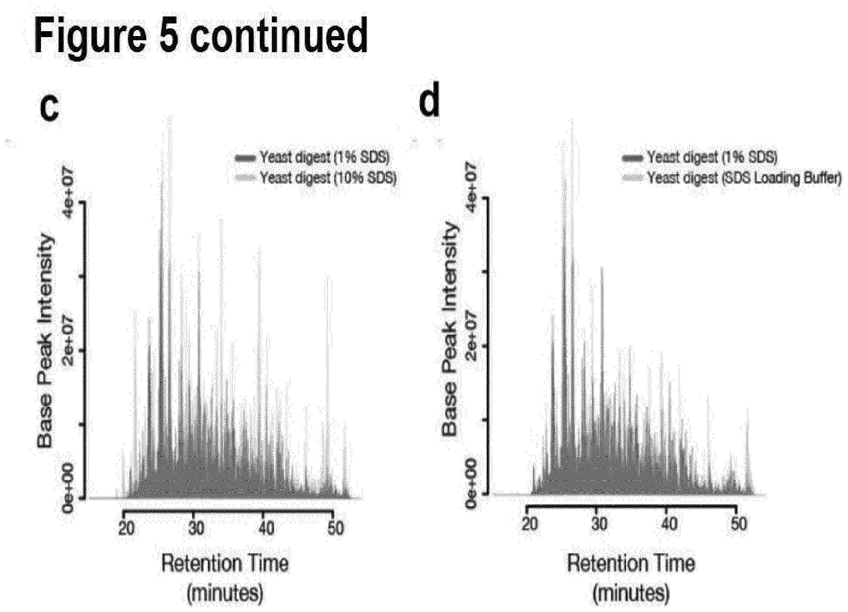

### Figure. 5: SP3 can enrich proteins in diverse conditions.

Proteins can be efficiently captured and rinsed using carboxylate modified paramagnetic beads in the SP3 protocol, in concentrated or dilute solution, or in the presence of detergents. Specified amounts of reduced and alkylated BSA in a 1% SDS buffer were used to determine binding capacity. Equivalent aliquots of a yeast cell lysate were used to determine recovery in dilute solutions, and in the presence of detergents. (a) BSA (10µg or 100µg) was combined with an equivalent amount of beads (1µg) and treated with SP3. Recovered protein was digested, and resulting peptides diluted to normalize the concentration between the samples based on the original starting amount prior to injection to the MS. (b) Equivalent amounts of yeast lysate were treated with SP3 directly, or after dilution to a total volume of 100µL. An equivalent volume of the recovered peptides was injected to the MS. (c) Yeast lysate containing 1% or 10% SDS (v/v) were treated with SP3. Mixtures were equivalently rinsed, digested, and injected to the MS. (d) Yeast lysate containing 1% SDS (v/v) or lx Laemmli buffer were treated with SP3. Mixtures were equivalently rinsed, digested, and injected to the MS.

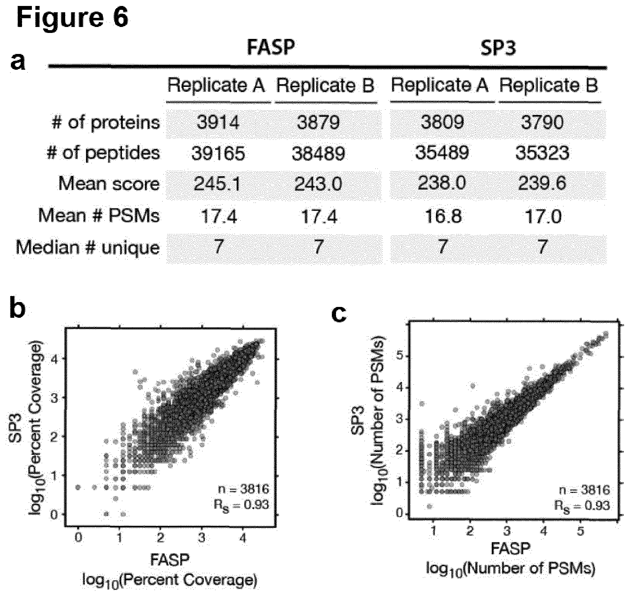

### Figure. 6: SP3 is equivalent to FASP for in-depth proteomics.

Duplicate aliquots of a yeast whole-cell lysate were prepared with SP3 or FASP prior to digestion with trypsin and rLysC. Peptides were fractionated with high-pH reversed phase chromatography and analyzed by MS. (a) Protein and peptide identification metrics from the FASP and SP3 data. (b) Scatter plot of the identity between the percent coverages assigned to matched proteins in the FASP and SP3 data. Data are represented on a logarithmic scale. (c) Scatter plot of the identity between the numbers of PSMs assigned to matched proteins in the FASP and SP3 data. Data are represented on a logarithmic scale.

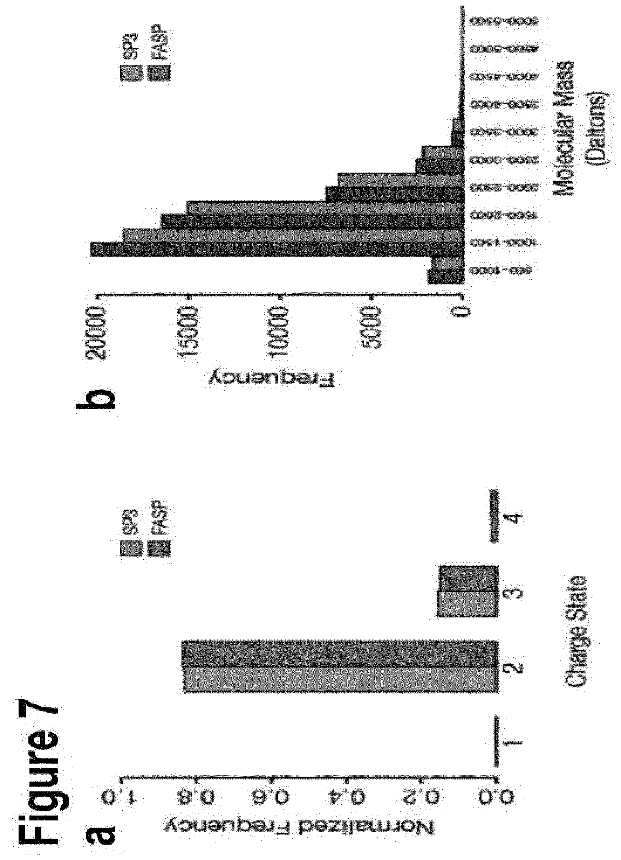

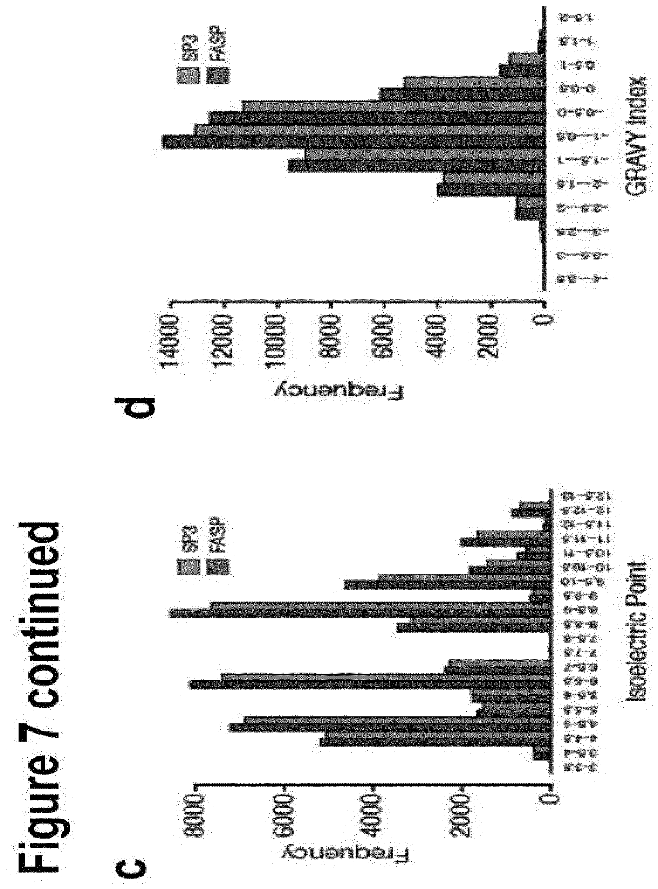

### Figure 7: SP3 and FASP peptide characteristics.

There is no observable bias in the peptides identified with SP3 when compared with those from the FASP approach. Duplicate aliquots of a yeast whole-cell lysate were prepared with SP3 or FASP prior to digestion with trypsin and rLysC. Peptides were fractionated with high-pH reversed phase chromatography and analyzed by MS. Plots represent histograms of charge state (a), molecular mass (b), isoelectric point (c), and GRAVY index (d) of identified unique peptides from each method. Peptide properties were determined using the ProtParam tool from ExPASy called from a Python script developed in-house.

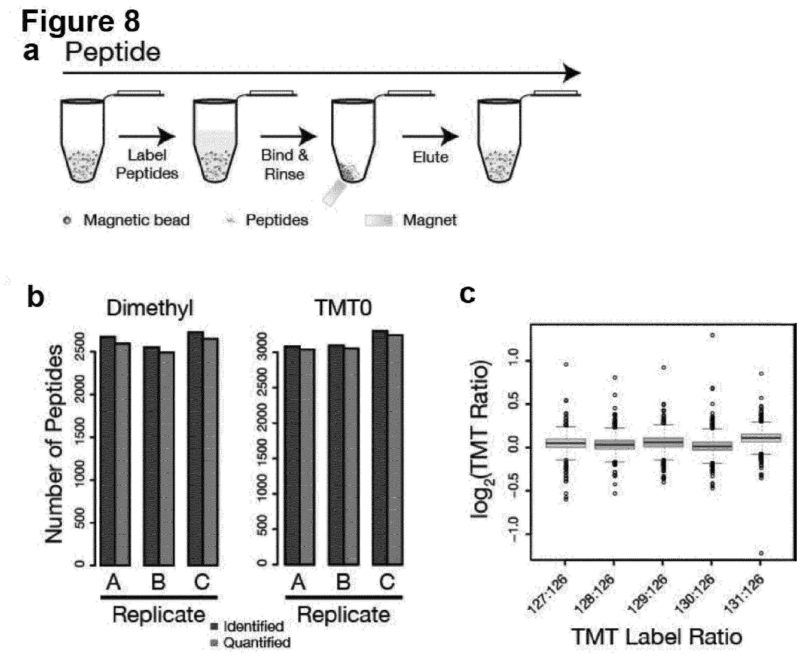

### Figure 8: Chemical labeling of peptides with SP3.

SP3 is compatible with chemical labeling of peptides using dimethyl or TMT approaches. Equivalent digests derived from a yeast-whole cell lysate were treated with a dimethyl to TMT labeling protocol in triplicate. (a) Chemical labeling is applied directly after SP3 protein clean-up in the same tube. SP3 peptide clean-up is performed with no modification and eluted prior to MS analysis. (b) Labeling efficiency of the dimethyl and TMT methods when coupled to SP3 as measured by the number of peptides identified with (quantified) or without (identified) the expected label in triplicate measurements. (c) Reproducibility of labeling as measured by deviance from an expected 1og2 fold change of 0. Replicate digests of a yeast lysate treated with the SP3 protocol were labeled in a TMT 6-plex reaction. Ratios represent protein-level calculations determined in PEAKS software.

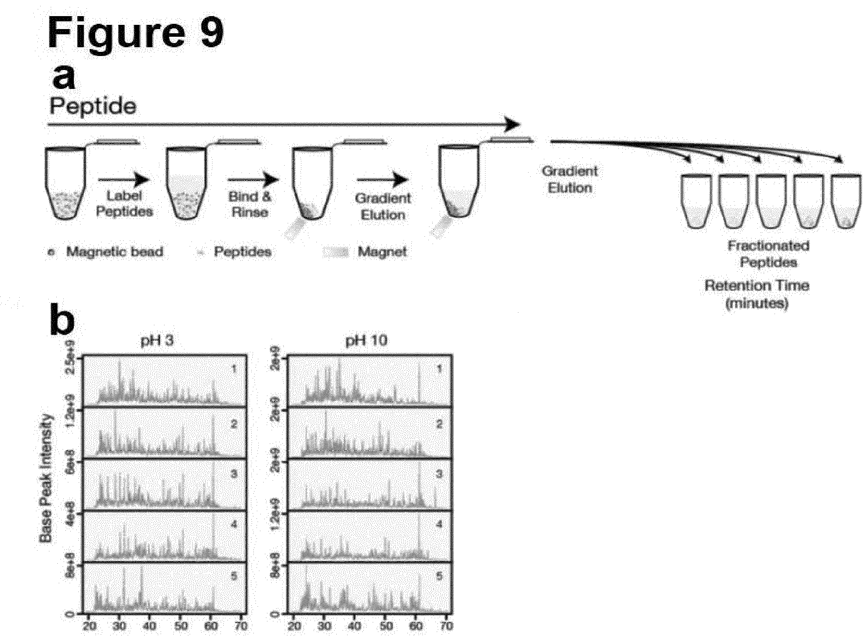

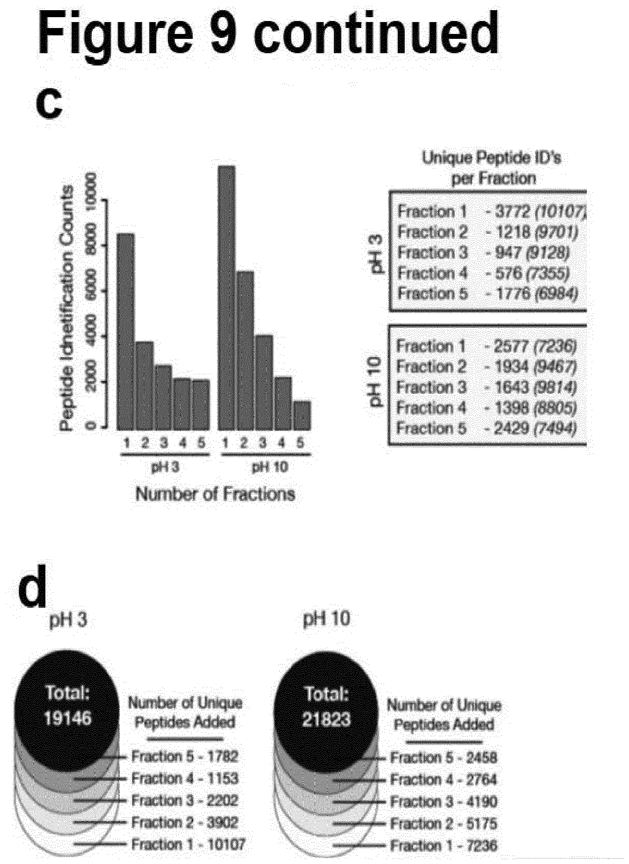

### Figure 9: Fractionation with SP3.

Peptides can be fractionated directly off of SP3 beads using HILIC and ERLIC-style conditions. 30 Equivalent digests of a yeast whole cell lysate were treated with peptide SP3. Peptides were eluted from the beads using a gradient of ACN at pH 3 (HILIC) or pH 10 (ERLIC). (a) Fractionation with SP3 is applied directly after peptide binding to the beads. Peptides are eluted through step-wise rinsing of the beads with a gradient of mobile phase. (b) Base peak chromatograms from fractionation and high and low pH. Numbers in the top right of the chromatograms represent the fraction number, with 1 being the first elution step. (c) Overlap between fractions as indicated by the number of fractions a peptide is successfully identified in. Values in tables represent the number of peptides completely unique to the listed fraction in the format: unique to fraction *(total number identified).* (d) Cumulative number of unique peptides identified through combination of each fraction beginning with 1. Total numbers in the top circle represent the sum of identified peptides through combination of all five fractions.

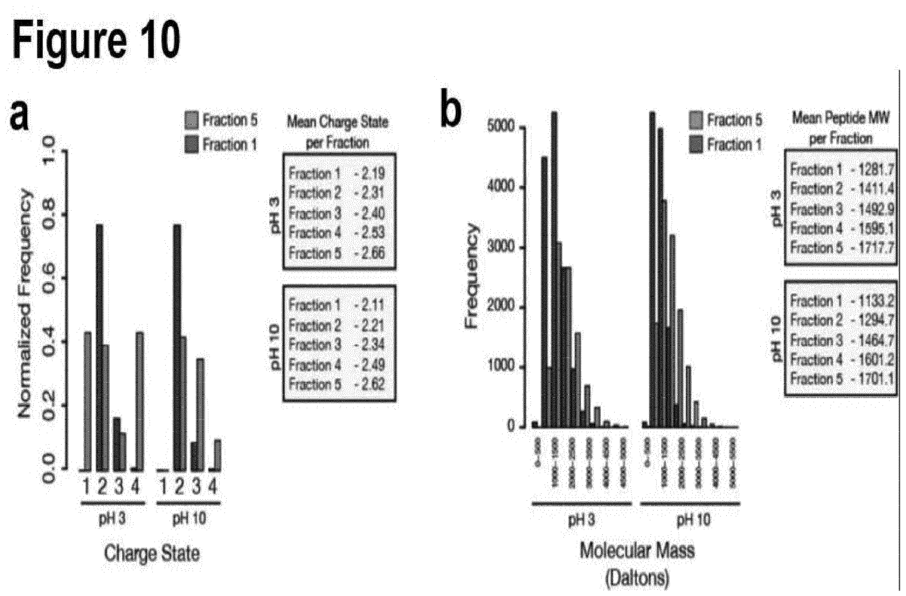

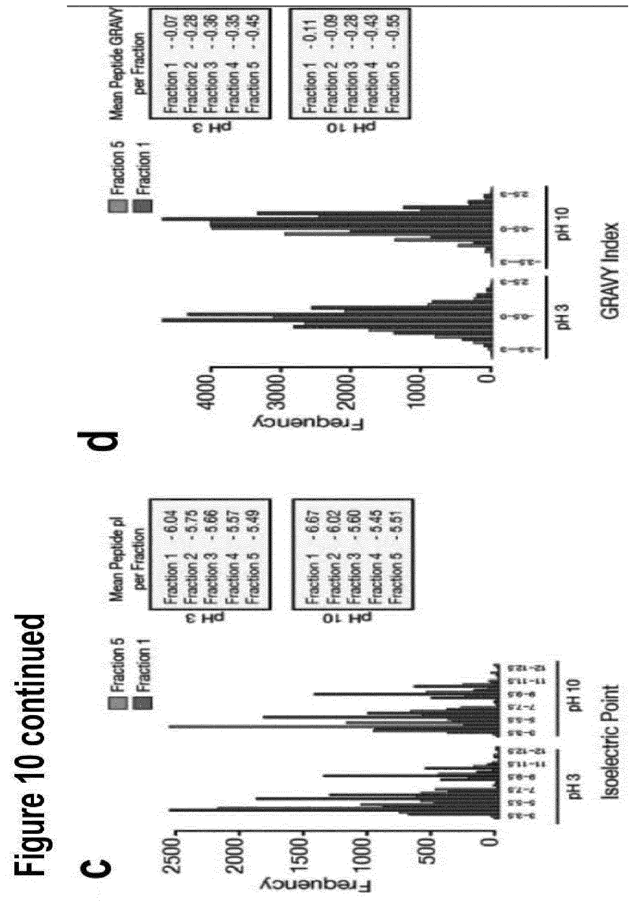

### Figure 10: Characteristics of fractionation with SP3.

Peptides identified after SP3 fractionation eluted based on the properties of HILIC or ERLIC. Properties of unique peptides identified in each fraction were determined using the ProtParam tool from ExPASy called from a Python script developed in-house. Plots represent histograms of charge state (a), molecular mass (b), isoelectric point (c), and GRAVY index (d). Tables on the right of each plot display mean values of all identified peptides for each fraction for the specific property of interest.

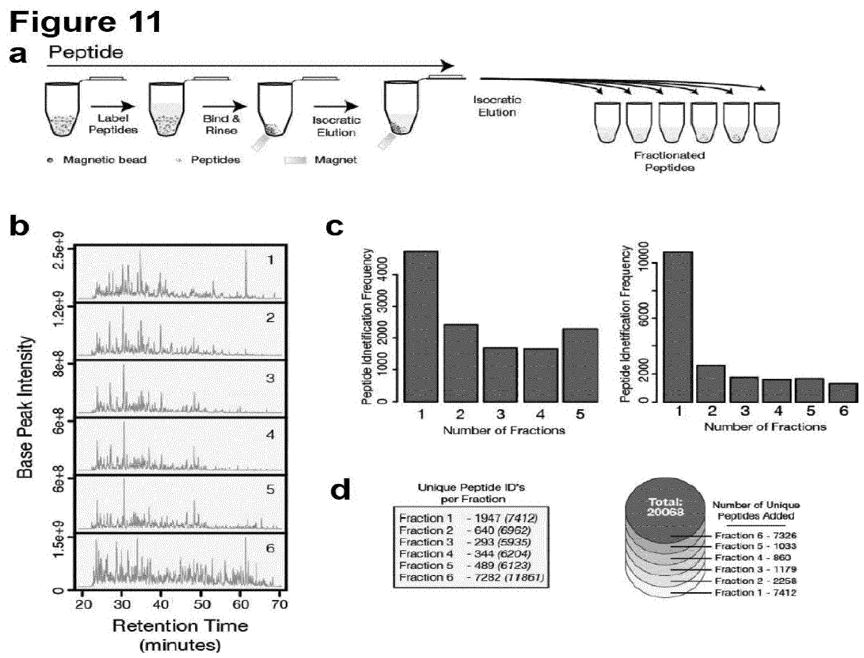

### Figure 11: Isocractic ERLIC fractionation with SP3.

Peptide elution in an isocratic mode at in high pH conditions is not as effective at fractionation of peptides compared with a gradient elution. Equivalent digests of a yeast whole cell lysate were treated with peptide SP3. Peptides were eluted from the beads using a repeated rinses of a 90% ACN with 10 mM formic acid solution at pH 10. (a) Elution is performed directly after binding of peptides to beads in SP3, into 6 separate fractions where the final step is a water elution. (b) Base peak chromatograms from fractionation in isocratic conditions. Numbers in the top right of the chromatograms represent the fraction number, with 1 being the first elution step. (c) Overlap between fractions as indicated by the number of fractions a peptide is successfully identified in. Separate plots are included to illustrate the high overlap between fractions 1 to 5, and the dissimilarity with fraction 6 (water elution). (d) Table displaying the number of peptides completely unique to the listed fraction in the format: unique to fraction *(total number identified).* Beside is the cumulative number of unique peptides identified through combination of each fraction beginning with 1. Total numbers in the top circle represent the sum of identified peptides through combination of all six fractions.

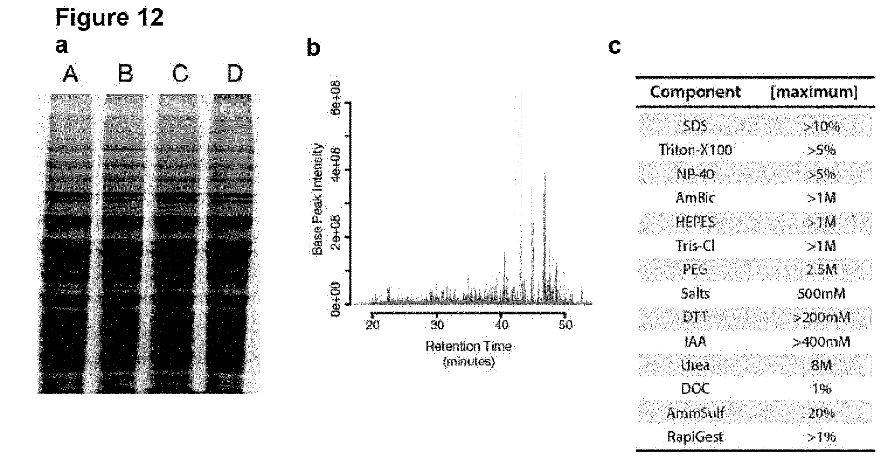

### Figure 12: Compatible materials and chemicals with SP3.

SP3 can be performed in a wide variety of conditions with beads from separate manufacturers. (a) Coomassie stained SDS-PAGE gel analysis of a yeast cell lysate (-5Oug of protein) that remained untreated (A), or was prepared with carboxylate beads from two separate manufacturers: Ampure XP from Beckman Coulter (B) and PCR Clean from CleanNA (C and D). The CleanNA beads are used in two separate conditions, with poly-ehtylene glycol (C) as a precipitation agent or ammonium sulfate (D). (b) MS base peak chromatograms from analysis of aliquots of a yeast cell lysate with two types of Sera-Mag beads. (c) Table of SP3 compatible concentrations of common reagents used in proteomics studies. A wide variety of rinses can be used in combination with SP3 to promote removal of contaminating substances (data not shown).

### Detailed Descriptions of the Invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Several documents are cited throughout the text of this specification. Each of the documents cited herein (including all patents, patent applications, scientific publications, manufacturer's specifications, instructions etc.), whether supra or infra, is hereby incorporated by reference in its entirety. Nothing herein is to be construed as an admission that the invention is not entitled to antedate such disclosure by virtue of prior invention. Some of the documents cited herein are characterized as being *"incorporated by reference".* In the event of a conflict between the definitions or teachings of such incorporated references and definitions or teachings recited in the present specification, the text of the present specification takes precedence.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

In the following, some definitions of terms frequently used in this specification are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

In the context of the present invention, the term "peptide" refers to a short polymer of amino acids linked by peptide bonds. It has the same chemical (peptide) bonds as proteins but is commonly shorter in length. The shortest peptide is a "dipetide" consisting of two amino acids joined by a peptide bond. There can also be tripeptides, tetrapeptides, pentapeptides etc. A peptide has an amino end and a carboxyl end, unless it is a cyclic peptide.

The term "polypeptide" refers to a single linear chain of amino acids bonded together by peptide bonds and preferably comprises at least five amino acids. A polypeptide can be one chain or may be composed of more than one chains, which are held together by covalent bonds, e.g. disulphide bonds and/or non-covalent bonds. Polypeptides that can be purified with the method of the invention preferably have a length of at least five amino acids, more preferably a length of at least 10 amino acids, 15 amino acids, 20 amino acids, 25 amino acids, 30 amino acids, 35 amino acids, 40 amino acids, 45 amino acids or 50 amino acids or longer.

As used in this specification the term "solid phase" is used to refer to the heterologous phase in contact with the solution comprising polypeptides. The solid phase allows immobilization of the negatively charged moieties. This can be effected by providing the solid phase with coupling groups that can form covalent bonds with the hydrophilic moieties to be attached. Preferably the solid phase includes solid phase particles, like microbeads, microparticles or microspheres. It is preferred that these solid phase particles are characterized by uniformity in size, preferably by an average diameter in the range from 0,5 to 1,5 µm, i.e. the average diameter can be 0,5; 0,6; 0,7; 0,8; 0,9; 1,0; 1,1; 1,2; 1,3; 1,4; 1,5 µm. Preferably, the solid phase comprises, essentially consists or consist of glass or an organic polymer. Organic polymers include but are not limited to polystyrene, polymethylstyrene, polyethylstyrene, and homologs thereof, polymethyl acrylate, polyethyl acrylate, polymethyl methacrylate, polyethyl methacrylate and homologs thereof, polymethylmetacrylatem carboxylate-modified polymethacrylate, and carboxylate-modified polystyrene and non-styrene materials.

The solid phase may also comprise a magnetic or paramagnetic substance, which allows the concentration of a solid phase heterologously dispersed in a polypeptide solution by a magnetic field. Preferably such magnetic or paramagnetic substance is located in the center of a solid phase particle and does not contact the polypeptide solution, e.g. due to a polymer coat which covers the magnetic or paramagnetic substance. Thus, magnetic or paramagnetic microbeads or microparticles are particularly preferred solid phases for use in the method or kit of the present invention. The solid phase particles preferably show colloidal stability in the absence of a magnetic field. The solid phase is, preferably stable in physiological and lysis buffer systems at a broad pH range. They can be sonicated and thermocycled at high temperatures (> 90°C), e.g. for the purpose of cell lysis.

The term "hydrophilic surface" refers to a surface to which either hydrophilic compounds are covalently or non-covalently attached or which is formed of a polymer that has hydrophilic properties. Preferably the polymer with hydrophilic properties is an organic polymer, preferred polymers are polyacrylamide, polyacrylic acid, polyacrylimide, polyelectrolytes, polyethylenimin, polyethylenglycol, polyethylenoxid, polyvinylalcohol, polyvinylpyrrolidon polystyrenesulfonic acid, copolymers of styrene and maleic acid, vinyl methyl ether malic acid copolymer, and polyvinylsulfonic acid. Preferred examples of hydrophilic compounds that are attached to a surface preferably an organic polymer surface comprise aminoethylmethacrylate, carbohydrate, cucurbit[n]uril hydrate, dimethylaminomethyl methacrylate, fumaric acid, maleic acid, methacrylic acid, isopropylacrylamid, itaconic acid, N-vinyl carbazole, 4-pentenoic acid, polyalkylene glycol diamine, pyrrol, t-butylaminoethyl methacrylate, undecylenic acid, vinyl acetic acid, and vinylpyrdidine.

The term "negatively charged moieties" as used herein refers to an organic or anorganic compound. Preferably the organic compound or the anorganic compound carries at least one negative charge. Examples of negatively charged organic compounds include but are not limited to carboxylic acids, preferably aliphatic, saturated monocarboxylic acids like acetic acid; aliphated unsaturated monocarboxylic acids like acrylic acid; aliphatic saturated dicarboxylic acids like oxalic acid, succinic acid; aliphatic saturated tricarboxylic acids like citric acid; aliphatic unsaturated dicarboxylic acids like fumaric acid, maleic acid; aromatic carboxylic acids like benzoic acid, salicylic acid; heterocyclic carboxylic acids like nicotinic acid, pyrrolidine-2-carboxylic acid; aliphatic unsaturated cyclic monocarboxylic acids like abietic acid; fatty acids like arachidonic acid, linoleic acid or oleic acid. Further, the negatively charged moieties include carboxylic acid derivatives such as esters, amides, amines, halides, anhydrides, hydrazides, thiocarbonic acids, peroxy carbonic acids, and hydroxamic acids. Negatively charged anorganic compounds include but are not limited to silica acid and derivatives thereof, sulfate, nitrate, phosphate, phosphoric acid, sulphuric acid, sulfonic acid, sulfurous acid, nitric acid, boric acid and their derivatives such as esters, amides, amines, halides, anhydrides, hydrazides. Furthermore, one or more negatively charged moieties can be immobilized to the surface of the solid phase via a linker. This provides an improved interaction between the negatively charged moieties and the polypeptides in the solution. Preferably the linker molecule can be a straight or branched C₁-C₂₀ carbohydrate, preferably alkyl, alkenyl or alkinyl. The negatively charged moiety may be synthesized in a one step reaction, but may also require additional reaction steps, e.g. a coupling step.

In the context of the present invention a "precipitation solution" is used to refer to a solution containing salts which have the ability to precipitate proteins or polypeptides. A precipitation solution comprises ions with high ionic strength which include but are not limited to ammonium, potassium, sodium, lithium, magnesium, fluoride, sulphate, hydrogenphosphate, acetate, chloride, nitrate, bromide, chloride, iodide and perchlorate and combinations thereof. Particularly preferred is a solution comprising ammonium ions. Ions with high ionic strength increase hydrophobic interactions between proteins or polypeptides and therefore change the solubility of such polypeptides or proteins. If the precipitation solution containing salts in a suitable concentration is exposed to a solution containing proteins or polypeptides aggregate formation starts and protein and polypeptide and precipitates are formed (Current Protocols in Protein Science, 1997). The concentration of the ions with high ionic strength in the precipitation solution is such that the concentration in the resulting mixture with the protein solution is sufficient to precipitate the proteins. It is understood by the skilled person that the end concentration after mixture is decisive and, thus the required starting concentration will be determined by the relative amounts of the precipitation solution and polypeptide solution mixed. In general it is preferred to use precipitation solutions with as high a concentration of the high ionic strength ions as possible to add as little volume as possible to the polypeptide solution. Accordingly, the concentration of the high ionic strength ion comprised in the precipitation solution is close to or at the saturation concentration at regular storage conditions, e.g. between 4°C to 30°C of the precipitation solution. Preferred precipitation solutions comprise 1 Owt% ammonium sulphate.

The term "dehydration solution" refers to a solution capable of dehydrating the polypeptide solution by binding to the water molecules hydrating the polypeptides in the solution. This removal of the water leads to aggregation/precipitation of the polypeptides. Preferably, the dehydration solution comprises, essentially comprises or consist of a polar organic solvent and/or a hygroscopic polymer. As set out above regarding the precipitation solution the concentration of the polar organic solvent and/or the hygroscopic polymer is determined by the concentration required for promoting interaction between the polypeptides and bead surface after mixing with the polypeptide solution and will vary with the relative amounts mixed in the method of the invention. Generally, it is preferred to use a dehydration solution with the maximum amount of polar organic solvent and/or hygroscopic polymer comprised therein, which will lead to a stable solution.

The term "polar organic solvent" as used herein means a carbohydrate that dissolves a chemically different liquid, solid or gaseous solute in a certain quantity, resulting in a solution that is soluble in a certain volume of solvent at a specified temperature and a dielectric constant (also referred to as "relative permittivity") at 25°C of at least 6, preferably of at least 10. The polar organic solvent is preferably aprotic or protic, more preferably the polar organic solvent is aprotic. Examples of polar aprotic solvents include but are not limited to acetone, tetrahydrofuran (THF), ethyl acetate, acetone, dichloromethane (DCM), dimethylformamide (DMF), dimethyl sulfoxide and propylene carbonate, a particularly preferred aprotic solvent is ACN. Examples of polar protic solvents include but are not limited to acetic acid, methanol, ethanol, n-propanol, isopropanol, n-butanol, and formic acid.

The term "hygroscopic polymer" as used in the present application refers to a carbohydrate polymer that is capable of binding water molecules by van der Waals interaction. Preferred examples of hygroscopic polymers comprise polyethyleneglycol (PEG), synthetic polyelectrolytes, semisynthetic polyelectrolytes, protamins, and naturally or non-naturally occurring polysaccharides (Current Protocols in Protein Science, 1997). Through the binding of water solubilizing the polypeptides in the solution hygroscopic polymers lead to a reversible precipitate of polypeptides at certain concentrations. Examples of synthetic polyelectrolytes include but are not limited to polyacrylic acid, vinyl polymers such as polyacrylate (PAA), polymethacrylate (PMA), acid salts (polyanions), polyethylenimine (PEI). Semisynthetic polyelectrolytes include but are not limited to carboxymethylcellulose (CMC), sulphated cellulose, hydroxypropylmethylcellulose (HPMC), diethylaminoethylcellulose (DEAE-C), and sulphated dextrans. Natural occurring polysaccharides include but are not limited to glycogen, heparin sulphate, chondroitin sulphate, alginates, chitin and hyaluronic acid.

The term "polypeptide concentration" as used in the present application describes the amount of polypeptide used for the studies present in this application and is typically indicated as ng per ml. The determination of the proteins or peptides can be performed by art known methods, including, e.g. Bradford-assay. In this protein determination assay the sample of interest (containing the protein or polypeptide solution) is mixed with a solution of Coomassie-Brilliant-Blue and the absorption is measured at a wavelength of 595nm.

The term "washing solution" as used in the present application refers to a solution which removes non-polypeptide compounds from the bound polypeptides without substantially dissolving the polypeptides. Preferably, the washing solution comprise, essentially consist of or consists of a polar organic solvent or a salt comprising at least on ion that increases hydrophilic interactions of polypeptides. The polar organic solvent is preferably aprotic or protic, more preferably protic. Examples of polar aprotic solvents include but are not limited to DCM, THF, ethyl acetate, acetone, DMF, ACN, DMSO, propylene carbonate. Examples of polar protic solvents include but are not limited to acetic acid, methanol, ethanol, n-propanol, isopropanol, n-butanol, and formic acid. The salt comprising ions with high ionic strength include but are not limited to ammonium, potassium, sodium, lithium, magnesium, fluoride, sulphate, hydrogenphosphate, acetate, chloride, nitrate, bromide, chloride, iodide and perchlorate and combinations thereof, preferably the ion is ammonium.

The term "protein denaturant" is used in the present application to denote a compound which is able to disrupt the tertiary and depending on the concentration also the secondary structure of a native polypeptide without changing the primary structure of the polypeptide. Examples include but are not limited to anionic detergents comprising carboxylates, sulfonates, sulfates preferably sodium dodecyl sulphate. (SDS); cationic detergents comprising quaternary ammonium compounds like cetyltrimethylammoniumbromid (CTAB); zwitterionic detergents comprising combinations of quaternary ammonium compounds and carboxylates; urea, guanidinium salts, salts of bile acids, like cholate or deoxycholate (DOC).

The term "labeling" as used in the present application refers to a method of attaching a detectable moiety to polypeptides or introducing a detectably moiety into the polypeptides. Preferred are labels, markers or tags which provide quantification of labeled polypeptides in mass spectrometry analysis. The method of stable isotope labelling entails replacing specific atoms of the polypeptides, e.g. C, O, N, or S, with their isotopes. This includes but is not limited to methods like stable isotope labeling by amino acids in cell culture (SILAC), trypsin-catalyzed ¹⁸O labeling, isotope coded affinity tagging (ICAT), isobaric tags for relative and absolute quantitation (iTRAQ). One common method in the field of stable isotope labeling is dimethyllabeling comprising a reagent for the generation of a Schiff base with a primary amine, a reducing agent and a suitable buffer. Another widespread method is called tandem mass tag (TMT) labeling comprising a label reagent, a suitable buffer, an organic solvent, a denaturating reagent, a reducing reagent, an alkylating reagent, a quenching reagent and a protease.

### Embodiments

In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. In the work leading to the present invention, it was surprisingly shown that polypeptides can aggregate onto a solid phase comprising a hydrophilic surface.

Based on these results the present invention provides in a first aspect a method of reversibly binding polypeptides to a solid phase comprising a hydrophilic surface, further comprising the step of contacting the solid phase, a solution containing the polypeptides and a dehydration solution and/or a precipitation solution.

In a preferred embodiment of the first aspect, the hydrophilic surface of the solid phase comprises or consists of a polymer that has hydrophilic properties. The term "hydrophilic" is used to refer to the tendency of a compound to interact with or be dissolved by water and other polar substances. Preferably the polymer with hydrophilic properties is an organic polymer. Preferred polymers are polyacrylamide, polyacrylic acid, polyacrylimide, polyelectrolytes, polyethylenimin, polyethylenglycol, polyethylenoxid, polyvinylalcohol, polyvinylpyrrolidon polystyrenesulfonic acid, copolymers of styrene and maleic acid, vinyl methyl ether malic acid copolymer, and polyvinylsulfonic acid. Preferred examples of hydrophilic compounds that are attached to a surface to form a hydrophilic surface comprise aminoethylmethacrylate, carbohydrate, cucurbit[n]uril hydrate, dimethylaminomethyl methacrylate, fumaric acid, maleic acid, methacrylic acid, isopropylacrylamid, itaconic acid, N-vinyl carbazole, 4-pentenoic acid, polyalkylene glycol diamine, pyrrol, t-butylaminoethyl methacrylate, undecylenic acid, vinyl acetic acid, and vinylpyrdidine

In a preferred embodiment of the present invention the salt or the salts are applied to the protein solution at a concentration which leads to a polypeptide precipitating concentration in the resulting mixture. Preferred salts are ammonium sulphate, ammonium chloride and sodium chloride. In a further preferred embodiment the concentration of ammonium, preferably ammonium sulphate in the final composition comprising the polypeptide is in the range from 195% saturated solution. The final concentration of sodium, preferably sodium chloride in the composition comprising the polypeptide ranges from 100 mM to 3 M, i.e. preferably 100 mM, 200 mM, 300 mM, 400 mM, 500 mM, 600 mM, 700 mM, 800 mM, 900 mM, 1 M, 1.2, 1.4, 1.5, 1.6, 1.8, 2.0, 2.2, 2.4, 2.6, 2.8 or 3.0 M.

The solution containing said polypeptides may also comprise a detergent. Detergents are typically used when preparing protein solutions to improve solubilisation of proteins, in particular lipophilic proteins. However, the presence of detergents often interferes with subsequent absorption of the proteins and, thus purification steps. For example, the absorption of proteins to surface-functionalized diamond nanocrystallites is significantly impaired, if the detergent concentrations in the protein solution exceeds 0.5% SDS (see, e.g. by Chen, W.-H. et al., 2006, Anal. Chem. 78: 4228). The tolerance against high detergent concentrations in the binding buffer is a further advantage of the method of the present invention. Thus, it is preferred that the solution containing a detergent comprises at least 1%, 1.5%, 2%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0% or more detergent. Preferred detergents that may be included in the solution comprising the proteins are ionic detergents, non-ionic detergents or mixtures thereof. Preferred detergents are selected from the group consisting of carboxylates, sulfonates, sulfates preferably sodium dodecyl sulphate (SDS); cationic detergents comprising quaternary ammonium compounds like cetyltrimethylammoniumbromid (CTAB); zwitterionic detergents comprising combinations of quaternary ammonium compounds and carboxylates; urea, guanidinium salts, salts of bile acids, like cholate or desoxycholat (DOC); and non-ionic detergents comprising polyoxypropylen-polyoxyethylen-block copolymers, e.g. Pluronic or tetrapolyols, or polysorbate detergents comprising polysorbate 20 (Tween^{®} 20) or detergents comprising polyoxethylen and a hydrophobic group, e.g. Triton X-100 or NP-40. If Triton^{®}-X100 is comprised in the protein solution it is preferred that the binding solution comprises more than 1.0% Triton^{®}-X100, more preferably more than 1.5% Triton^{®}-X100, even more preferably more than 2.0% Triton^{®}-X100, more than 3.0% Triton^{®}-X100, more than 4.0% Triton^{®}-X100, and most preferably 5.0% or more Triton X-100. If NP-40 is comprised in the protein solution it is preferred that the binding solution comprises more than 1.0% NP-40, more preferably more than 1.5% NP-40, even more preferably more than 2.0% NP-40, more than 3.0% NP-40, more than 4.0% NP-40, and most preferably 5.0% or more NP-40. If DOC is comprised in the protein solution it is preferred that the binding solution comprises more than 0.2% DOC, more preferably more than 0.4% DOC, even more preferably more than 0.6% DOC, more than 0.8%, and most preferably 1.0% or more DOC. Most preferably the protein solution used in step a) of the method of the present invention comprises more than 2.0%, more preferably more than 2.5% SDS, even more preferably more than 3.0% SDS, more than 4%, more than 5%, more than 6%, more than 7.0% more than 8.0%, more than 9.0% and most preferably 10% or more SDS.

The binding properties of the proteins in the solution will also depend on the pH of the solution. For maximum recovery, a solution pH value of 3 should be used to maximize the hydrophilicity of proteins and tryptic peptides. In instances where fractionation off-bead is desired, a pH value of 10 should be used to promote electrostatic repulsion from the beads. Thus, is preferred that the solution comprises a buffer that keeps the pH at the desired value, preferably the pH of the solution is 3, 3.5, 4, 4.5, 5, 5.5, 6, 6.5, 7, 7.5, 8, 8.5, 9, 9.5 or 10.

Preferably, the polar organic solvent is added to the composition at a final concentration of 60% (v/v) for polypeptides, more preferably at a final concentration of 40% (v/v) and even more preferably at a final concentration of 50% (v/v). Preferably, the polar organic solvent is added to the composition at a final concentration of 90% (v/v) for peptides, more preferably at a final concentration of 95% (v/v).

In a further preferred embodiment of the first aspect of the invention the dehydration solution optionally comprises a hygroscopic polymer, which is used for the precipitation of proteins or polypeptides, preferably polyethylenglycole. More preferably, the polyethylenglycole used possesses a molecular weight in the range between 4000 to 8000Da. It is added to the composition to lead to a final concentration in the range of 1 to 30%.

Once precipitated and/or bounded onto the solid phase the polypeptide precipitated and/or bounded polypeptides are washed once or more with suitable washing solutions. These washing solutions may be identical or of different compositions. Washing is typically carried out by separating the solid phase from the solution and removing the solution as completely as possible and applying the washing solution to the solid phase. If the solid phase comprises solid particles the particles are resuspended in the washing solution. The washing solutions may comprise above indicated components of the protein solution, e.g. detergents and salts, in the same or higher concentrations as indicated above.

In a preferred embodiment of the present invention the "washing solution" is ethanol. More preferably in a final concentration of 70% (v/v) and acetonitrile in a final concentration of up to 100% is used.

In an embodiment in which the washing solution comprises a salt it is preferred that the salt concentration in the washing solution ranges from 1 to 3 M, i.e. 1, 1.5, 2.0, 2.5, 3.0. It is particularly preferred that the salt is sodium chloride, more preferably 2.0 to 2.5 M sodium chloride. An increase in the salt concentration increases the solubilisation of contaminants that may adhere to the proteins.

In a further preferred embodiment the washing solution comprises a chaotrope or a salt comprising at least one ion that increases hydrophobic interaction of polypeptides, preferably in a concentration range from 1 M to 8 M, preferably 1, 1.5, 2.0, 2.5, 3.0, 3.5, 4.0, 4.5, 5.0, 5.5, 6.0, 6.5, 7.0, 7.5 or 8.0. In an embodiment in which the washing solution comprises a chaotrope it is preferred that this chaotrope is urea. Particularly preferred amounts of urea comprised in the washing solution are between the ranges of 6 to 9 M, preferably 8 M.

In a further preferred embodiment the washing solution optionally comprises a protein denaturant, a detergent or a salt, which aid in removing non-polypeptide components from the polypeptide precipitate. Since most denaturants and detergents (e.g. SDS) are incompatible with enzymatic digestions and downstream MS-analysis, it is preferred that these are removed prior to elution or further use of the precipitated and/or bounded polypeptides. To that end at least the last washing step is carried out in the absence of detergent and/or denaturant to remove residual denaturant and/or detergent from the precipitate. Although ultrafiltration (FASP; Wisniewski, J.R. *et al.,* 2009) or bead-based protocols (Hengel, S.M. *et al.,* 2012; Bereman, M.S. *et al.,* 2011) are efficient for removal of detergents, they have minimal flexibility and require significant sample handling (filtration, centrifugation, concentration), reducing their overall effciency. Thus, the present invention provides an easy way of removing denaturants and/or detergents from the reversibly bound polypeptides. Accordingly the precipitation protocol of the present invention is compatible with the use of a SDS-containing cell lysate and digestion of the eluted polypeptides and subsequent MS-analysis. The precipitation protocol of the present invention lead to enhanced efficiency in polypeptide recovery when compared with standard protocols for detergent removal. Furthermore the method of the present invention leads to an increase in MS peptide intensity, i.e. enhanced recovery, and the number of proteins identified.

The "aqueous elution solution" used in the context of the method of the present invention comprises water in an amount that is sufficient to redissolve the majority of precipitated and/or bounded polypeptides, preferably at least 80%, more preferably at least 85% and even more preferably at least 90% of the precipitated and/or bounded polypeptides. The aqueous elution solution, preferably comprises water and salts/acids/bases providing a buffer system such as but not limited to phosphate buffered saline (PBS), 4-2-hydroxyethyl-1-piperazineethanesulfonic acid(HEPES), potassium based buffer (KC1), tris(hydroxymethyl)methylamine (TRIS), 2-(N-morpholino)ethanesulfonic acid (MES), 3-(N-morpholino)propanesulfonic acid (MOPS), piperazine-N,N'-bis(2-ethanesulfonic acid) (PIPES), saline sodium citrate (SSC), N-tris(hydroxymethyl)methylglycine (tricine), 3 - {[tris(hydroxymethyl)methyl] amino}propanesulfonic acid (TAPS), N,N-bis(2-hydroxyethyl)glycine (bicine), 3- [N-Tris(hydroxymethyl)methylamino] -2-hydroxyprop ane sulfonic Acid (TAPSO), 2-{[tris(hydroxymethyl)methyll amino } ethanesulfonic acid (TES), and dimethylarsinic acid (cacodylate).

It is preferred that the pH is between 5 and 10, preferably 5, 5.5, 6, 6.5, 7, 7.5, 8, 8.5, 9, 9.5 or 10. More preferably, for maximum recovery, a solution pH value of 8 should be used to minimize the hydrophilicity of the proteins and peptides to promote elution.

In this embodiment it is preferred that a protease is added to the "aqueous elution solution", more preferably trypsin or LysC. The addition of a protease allows digestion of the polypeptides in the presence of the solid phase. Afterwards they can again be precipitated and/or bounded bound onto the solid phase, which allows another purification step, e.g. before subjecting the polypeptide digests to MS analysis. To that end the dehydration or precipitation solution described above can be added to the beads and the aqueous elution solution containing the digested polypeptides.

If magnetic beads are used the beads can be concentrated with a magnetic field, rinsed and subsequently eluted upon addition of the aqueous elution solution. In this case the entire workflow of purification only requires 15 minutes, representing a significant improvement in throughput compared to other established workflow protocols (e.g. FASP).

In a further embodiment of the method of the invention the aqueous elution solution containing the (digested) polypeptides can be subjected to mass spectrometry for identification, characterisation, quantification, purification, concentration and/or separation of polypeptides without further steps of sample preparation. Though obtaining reliable, deep proteome coverage remains a central goal in many proteomics analyses, this task is challenging for samples where a limited amount of material is available (Altelaar, A. *et al.,* 2012). These analyses are complicated by losses during sample handling steps present in virtually all proteomic workflows. Since the entire method of the invention for polypeptide enrichment can be performed in a single tube without filtering, precipitation or resolubilisation steps, it facilitates efficient analyses of cell-limited samples.

The high recovery of polypeptides renders the method of the invention suitable for in-depth proteome studies by comparative analysis with FASP. In addition, there was no observable bias related to protein enrichment. Thus, the method of the present invention is amenable to the generation of samples where MS-analysis can yield deep and reproducible proteome coverage. This affords in-depth proteome characterization and quantification even of small sample sizes, promising that the invention may find its place in clinical applications.

In a further embodiment of the present invention polypeptides can be labeled for mass spectrometry analysis. Preferably, the labelling is carried out after the polypeptides are reversibly bound, washed and released, i.e. purified. A preferred method of labelling is stable isotopic labeling. More preferably labeling reagents are selected from the group comprising components suitable for dimethyllabeling like a reagent for the generation of a Schiff base with a primary amine, more preferably a 1% formaldehyde solution; a reducing agent, more preferably cyanoborohydride solution 155mM; a suitable buffer, more preferably 2-(4-(2-hdroxyethyl)-1-piperazinyl)-ethansulfonic acid (HEPES) 50mM, pH 8 with 1% deoxycholic acid (DOC). In another preferred embodiment of the sixth aspect the peptides are further treated by tandem mass tag labeling comprising a label reagent, a suitable buffer, preferably triethylammoniumbicarbonate buffer in a 1M concentration; an organic solvent, preferably acetonitrile; a denaturating reagent, preferably a 10% SDS solution; a reducing reagent, preferably tris(2-carboxyethyl)-phosphine (TCEP); an alkylating reagent, preferably iodoacetamide; a quenching reagent, preferably a 50% hydroxylamine solution and a protease, preferably tryp sin.

In a second aspect the present invention relates to a kit comprising:
(i) a solid phase comprising a hydrophilic surface , and
(ii) a dehydration solution and/or a precipitation solution.

In the context of the second aspect of the invention all reagents described in detail above in the context of the method of the present invention can also be included in the kit of the present invention.

In a preferred embodiment the kit further comprises:
(i) reagents for labeling polypeptides, preferably including stable isotopic labeling components;
(ii) reagents for eluting polypeptides, preferably aqueous buffers;
(iii) reagents for digestion of polypeptides, preferably proteases and detergents;
(iv) suitable reagents for fractionation of polypeptides;
(v) reagents for determining protein concentration;
(vi) suitable reagents for glycopeptide or phosphopeptide enrichment, and/or
(vii) suitable reagents for cell lysis.

In a preferred embodiment the reagents for labeling polypeptides are selected from the group consisting of:
(i) components suitable for dimethyllabeling, preferably a reagent for the generation of a Schiff base with a primary amine, a reducing agent, a suitable buffer and/or
(ii) components suitable for chemical isobaric mass tag labeling preferably a label reagent, a suitable buffer, an organic solvent, a denaturating reagent, a reducing reagent, an alkylating reagent, a quenching reagent and a protease and/or
(iii) components suitable for neutron-encoded chemical labelling.

In a third aspect the present invention comprises the use of a solid phase comprising a hydrophilic surface or a kit of the present invention for the analysis, identification, characterisation, quantification, purification, concentration and/or separation of polypeptides.

Furthermore, the present invention provides the following items:
1. A method of reversibly binding polypeptides to a solid phase comprising a hydrophilic surface , comprising the step:
   a) of contacting said solid phase, a solution containing said polypeptides; and a dehydration solution and/or a precipitation solution.
2. The method of item 1, wherein the hydrophilic surface is a polymer with hydrophilic properties, preferably polyacrylamide, polyacrylic acid, polyacrylimide, polyelectrolytes, polyethylenimin, polyethylenglycol, polyethylenoxid, polyvinylalcohol, polyvinylpyrrolidon polystyrenesulfonic acid, copolymers of styrene and maleic acid, vinyl methyl ether malic acid copolymer, and polyvinylsulfonic acid or comprises a hydrophilic compound selected from the group consisting of aminoethylmethacrylate, carbohydrate, cucurbit[n]uril hydrate, dimethylaminomethyl methacrylate, fumaric acid, maleic acid, methacrylic acid, isopropylacrylamid, itaconic acid, N-vinyl carbazole, 4-pentenoic acid, polyalkylene glycol diamine, pyrrol, t-butylaminoethyl methacrylate, undecylenic acid, vinyl acetic acid, and vinylpyrdidine.
3. The method of item 1 or 2, wherein the hydrophilic surface further comprises a negatively charged moiety selected from the group comprising a carboxylic acid moiety, silica moiety, a sulphate moiety, a phosphate moiety, nitrite moiety, nitrate moiety.
4. The method of any of items 1 to 3, wherein the solid phase comprises microbeads or microparticles.
5. The method of any of items 1 to 4, wherein the precipitations solution comprises salt, wherein the salt comprises at least one ion that increases hydrophobic interaction of polypeptides.
6. The method of item 5, wherein the ions are selected from the group consisting of ammonium, potassium, sodium, lithium, magnesium, fluoride, sulphate, hydrogenphosphate, acetate, chloride, nitrate, bromide, chlorate, iodide, and perchlorate, preferably ammonium sulphate, ammonium acetate, potassium chloride, lithium chloride, sodium chloride, magnesium sulphate, calcium sulphate, sodium perchlorate or combinations thereof.
7. The method of any of item 5 or 6, wherein the salt(s) is (are) comprised in the composition of step a) at a polypeptide precipitating concentration.
8. The method of item 1, wherein the dehydration solution comprises a polar organic solvent and/or a hygroscopic polymer.
9. The method of item 8, wherein the polar aprotic organic solvent is selected from the group consisting of acetonitrile, acetone, alcohol, dichloromethane, dimethylformamide (DMF), dimethylsulfoxide (DMSO), ethylacetate, hexamethylphosphoramide (HPMA), and tetrahydrofuran (THF).
10. The method of item 9, wherein the alcohol is selected form the group consisting of ethanol, n-propanol, iso-propanol, n-butanol, sec-butanol, tert-butanol and pentanol.
11. The method of item 8, wherein the hygroscopic polymer is selected from the group consisting of polyethylene glycol, dextranes, alginates, cellulose, polyacrylic acid, tannic acid, and glycogen.
12. The method of item 11, wherein the polyethylene glycol has a molecular weight in the range of 4000 to 8000 Da.
13. The method of any of items 1 to 12, wherein the dehydration solution and/or precipitation solution is added in an amount resulting in the reversible binding of at least 90% of the polypeptides in said solution.
14. The method of any of items 1 to 13, wherein one or more negatively charged carbohydrate moieties are immobilized via a linker.
15. The method of item 14, wherein the linker is straight or branched C₁-C₂₀-residue, optionally comprising one or more heteroatoms.
16. The method of any of items 1 to 15, wherein the solution containing said polypeptides is selected from a whole cell extract, a whole cell extract digest, proteins derived from tissue, recombinant purified proteins, purified proteins, a protein digest, a purified protein digest, and a peptide library.
17. The method of any of items 1 to 16, wherein the solid phase comprising reversibly bound polypeptides is separated from the solution.
18. The method of item 17, wherein the solid phase is washed with a washing solution comprising a polar organic solvent or a salt or chaotrope comprising at least one ion that increases hydrophobic interaction of polypeptides, preferably in a concentration range from 1M to 8M.
19. The method of item 18, wherein the polar organic solvent is selected from the group consisting of alcohol, preferably ethanol, acetonitrile, trifluoroethanol, dichloromethane, dimethylformamide, and dimethylsulfoxide.
20. The method of item 18, wherein the washing solution comprises one or more further components selected from the group consisting of a protein denaturant, a detergent or a salt.
21. The method of any of items 1 to 20, comprising the further step of adding a protease.
22. The method of any of items 1 to 21, wherein the polypeptides are released from the solid phase with an aqueous elution solution.
23. The method of item 22, comprising the further step of adding a protease to the aqueous solution.
24. The method item 22 or 23, comprising the further step of adding a dehydration solution and/or a precipitation solution to the aqueous polypeptide solution.
25. The method of any of items 21 to 24, comprising the further step of submitting the aqueous solution to mass spectrometry.
26. Kit comprising:
   (i) a solid phase comprising a hydrophilic surface , and
   (ii) a dehydration solution and/or a precipitation solution.
27. Kit of item 26, further comprising:
   (i) reagents for labeling polypeptides, preferably including stable isotopic labeling components;
   (ii) reagents for eluting polypeptides, preferably aqueous buffers;
   (iii) reagents for digestion of polypeptides, preferably proteases and detergents;
   (iv) suitable reagents for fractionation of polypeptides;
   (v) reagents for determining protein concentration;
   (vi) suitable reagents for glycopeptide or phosphopeptide enrichment and/or
   (vii) suitable reagents for cell lysis.
28. Kit of item 27, wherein the reagents for labeling polypeptides are selected from the group consisting of:
   (i) components suitable for dimethyllabeling, preferably a reagent for the generation of a Schiff base with a primary amine, a reducing agent, a suitable buffer; and/or
   (ii) components suitable for chemical isobaric mass tag labeling preferably a label reagent, a suitable buffer, an organic solvent, a denaturating reagent, a reducing
      reagent, an alkylating reagent, a quenching reagent and a protease; and/or
   (iii) components suitable for neutron-encoded chemical labelling.
29. Use of a solid phase comprising a hydrophilic surface or a kit of any of items 26 to 28, for the analysis, identification, characterisation, quantification, purification, concentration and/or separation of polypeptides.

### Examples

### Example 1: Bead Preparation

In all experiments with SP3 we utilize commercially available beads that carry a carboxylate moiety. We have tested and verified the protocols used in this study with beads from Beckman Coulter (Ampure XP, CAT# A63880), CleanNA (Clean PCR, CAT# CPCR1300), and Thermo Fisher (Sera-Mag Speed Beads, CAT# 09-981-121, 09-981-123) (Supplementary Figure 9). In all cases, beads are an average diameter of lum and are coated with a hydrophilic surface. For all SP3 experiments in this manuscript a 1:1 combination mix of the two types of Sera-Mag speed beads is used. Beads are rinsed with water prior to use and stored at 4°C. Magnetic racks used in all experiments were prepared in-house

### Example 2: Cell Culture.

The yeast strain YAL6B (MATa, his3Δ leu2Δ met15 Δ lysl::KanMX6 arg4::KanMX4)' was cultured in rich medium (YPD) for all experiments. Replicate cultures were harvested at an optical density (OD600) of - 0.8. Cells were harvested through centrifugation, rinsed with ice-cold PBS and snap frozen until use. HeLa Kyoto cells were cultured in DMEM with Glutamax supplemented with 10% fetal bovine serum and lx non-essential amino acids. Cells were cultured at 37°C in a 5% CO₂ environment. Cells were harvested through incubation with a solution of 0.05% trypsin-EDTA, and centrifuged. Recovered cells were rinsed and counted prior to aliquoting to the desired cell number per tube. Approximate cell counts were acquired using a haemocytometer. Cell pellets were always directly lysed with no snap freezing. All cell culture reagents were obtained from Life Technologies unless noted otherwise.

### Example 3: Sample preparation and FASP (Filter assisted separation protocol)

Frozen yeast cell pellets were lysed using a combination of mechanical and solution based disruption approaches. Lysis buffer was composed of 1% SDS (Bio-Rad), 5mM dithiothreitol (DTT) (Bio-Rad), lx Complete Protease Inhibitor Cocktail-EDTA (Roche), prepared in 50mM HEPES buffer at pH 8.5 (Sigma). A pellet equivalent to 50m1 of - 0.8 **OD** 600 cells was combined with 500µ1 of lysis buffer in a 2m1 screw-cap tube containing a 500µ1 equivalent of acid-washed glass beads (Sigma, 425 to 600µm, CAT# G8772). Tubes were incubated for 5 minutes at 95°C and subsequently placed on ice for 5 minutes. Tubes were shaken vigorously on a Fast-Prep instrument (MP Biomedical) for 45 seconds at speed 5. Vials were then sonicated in a Bioruptor (Diagenode) for 15 cycles (30 seconds on, 30 seconds off) on the setting 'high' with chilling set at 4°C. Lysates were then recovered into 2m1 tubes by piercing the bottom of the screw cap tube with a heated syringe and centrifuging for 1 minute at 3,000g. Cell debris was pelleted with further centrifugation at 15,000g for 10 minutes. Recovered lysates were stored at -80°C until use. HeLa cells were lysed using a solution-based procedure without mechanical disruption. Specific numbers of harvested cells were diluted in phosphate buffered saline (PBS) such that the concentration did not exceed 25,000 cells in 5µ1. Lysis buffer was composed of 1% SDS (Bio-Rad), lx COmplete Protease Inhibitor Cocktail-EDTA (Roche), prepared in 50mM HEPES buffer at pH 8.5 (Sigma). Lysis was induced through addition of an equal volume of lysis buffer. Mixtures were heated for 5 minutes at 95°C and subsequently placed on ice for 5 minutes. To each tube, 25 Units of Benzonase Nuclease (Novagen, CAT# 70664) was added per 500,000 cells to degrade chromatin. Tubes were incubated at 37°C for 30 minutes in a thermocycler. This is a critical step in the lysis protocol, as excess chromatin in the lysate will disrupt the SP3 protocol. Proteins in the yeast, HeLa, and drosophila solutions were reduced through the addition of 5µ1 of 200mM DTT (Bio-Rad) per 100µ1 of lysate. Tubes were incubated at 45°C for 30 minutes in a thermocycler to facilitate disulfide reduction. Alkylation was performed through the addition of 10µ1 of 400mM iodoacetamide (IAA) per 100µ1 of lysate. Tubes were incubated at 24°C in the dark for 30 minutes in a thermocycler. Reactions were quenched through the addition of 10µ1 of 200mM DTT per 100µ1 of lysate. FASP was carried out as described previously with minor modifications (Wisniewski, J., Zougman, A., Nagaraj, N. & Mann, M. Universal sample preparation method for proteome analysis. Nat. Methods 6, 3 - 7, 2009). Briefly, each reduced and alkylated protein mixture was diluted to a final volume of 30µl with 50mM HEPES at pH 8. To this, 200µ1 of 8M urea was added to the protein sample and centrifuged in Vivacon (Sartorius) 30 KDa molecular weight cut-off filters for 10 minutes at 14,000g. This step was repeated a further 3 times. Filters were then rinsed 3 times with 100uL of 50mM HEPES, pH 8 by centrifugation. Proteolysis was carried out overnight in-filter in a wet-chamber at 37°C with a mixture of trypsin of rLysC at an enzyme to substrate ratio of 1:25. Peptides were eluted with sequential rinses of 50mM HEPES (2 times) and 200mM NaC1 (1 time). Eluted peptides were desalted using SepPaks (Waters) prior to MS analysis.

### Example 4: SP3 Optimization: Proteins

The Sera-Mag SP3 bead mix that had been rinsed was diluted to a final working concentration of 10µg/µl. To each protein mixture to be treated, 2µ1 of this bead mixture was added and mixed to generate a homogeneous solution. In the optimization methods where bovine serum albumin (BSA) was used, a stock solution of 50mg/ml was prepared in 1% SDS (Bio-Rad), 1 x Complete Protease Inhibitor Cocktail-EDTA (Roche), prepared in 50mM HEPES buffer at pH 8.5 (Sigma). The BSA was heated at 95°C for 5 minutes, placed on ice, reduced with DTT and alkylated with IAA as above. In the optimization methods where the yeast whole-cell lysate was used, 5uL (10µg of protein) of the reduced and alkylated mixture was utilized in all cases. To determine the optimal conditions for protein binding, the yeast whole-cell lysate was treated with a range of conditions. In control samples equivalent amounts of lysate were run directly with SDS-PAGE or treated with FASP. To determine the optimal environment for binding, the yeast lysate (10µg of protein) was mixed with 2µ1 of a 10µg/µl bead stock and treated with one of the following conditions: 1. An equal volume of a 5% formic acid solution was added to acidify the mixture (nanodiamond conditions), 2. A pre-determined volume of 100% acetonitrile was added to achieve a specific final percentage solution, 3. A small volume of 5% formic acid in combination with a pre-determined volume of 100% acetonitrile was added to achieve a specific final percentage solution, 4. An equivalent volume of a 2.5M NaC1, 20% ammonium sulfate solution was added (SPRI conditions). The rinsing conditions in SP3 are the same as those discussed below. In conditions 2 and 3, a specific concentration of acetonitrile must be reached. To determine these levels, recovery of the yeast lysate across a range of acetonitrile concentrations was tested (20 to 95%). As readout of recovery in these experiments, proteins eluted off of beads were directly digested and run on the MS. The signal intensity and complexity between runs was compared to determine the optimum percentage of acetonitrile. These curves were completed both in the presence and absence of formic acid (data not shown). A final concentration of 50% of acetonitrile in acidic conditions was found to give the highest and most reproducible recovery. To determine the binding capacity, lug of beads from a 10ug/uL stock was prepared with 1µg, 100µg, 200µg, and 400µg of reduced and alkylated BSA in SDS containing lysis buffer. The mixtures were treated with SP3 using 50% acetonitrile under acidic conditions. The recovered protein was digested and resulting peptides diluted such that the concentration was equal based on the starting amount of BSA. Recovery was measured using MS intensity and peak matching between samples. To test binding in the presence of contaminating substances, reduced and alkylated yeast lysates were used. Prior to treatment with SP3, lysates were spiked with the specific contaminating substance (e.g. 10% SDS). Recovered proteins were digested and the resulting peptides injected to the MS. Recovery was compared using MS intensity and chromatogram complexity between samples.

### Example 5: SP3 protocol: Proteins

The Sera-Mag SP3 bead mix that had been rinsed was diluted to a final working concentration of 10µg/µ1. To each protein mixture to be treated, 2µL of this bead mixture was added and mixed to generate a homogeneous solution. Unless noted otherwise, protein mixtures were adjusted to 50% acetonitrile under acidic conditions in all samples. Bead-protein solutions were mixed to ensure a homogeneous distribution of the beads and incubated for a total of 8 minutes at room temperature. After incubation, tubes were placed on a magnetic rack for 2 minutes. While on the magnet, the supernatant was removed and discarded. The beads were rinsed through addition of 200µ1 of 70% absolute ethanol. This step was repeated one further time, and in both cases the ethanol was discarded. Beads were then rinsed one further time with 180uL of 100% acetonitrile, and the supernatant discarded. All rinses were carried out on the magnetic rack. Rinsed beads were reconstituted in aqueous buffer to elute proteins dependent on the downstream experiment.

### Example 6: SP3 Optimization: Peptides

The Sera-Mag SP3 bead mix that had been rinsed was diluted to a final working concentration of 10µg/µ1. To each peptide mixture to be treated, 2µ1 of this bead mixture was added and mixed to generate a homogeneous solution. To determine the optimal conditions for protein binding, aliquots of derived from a single digest of the yeast whole-cell lysate were treated with a range of conditions. In control samples equivalent amounts of lysate were run directly with SDS-PAGE or treated with FASP. To determine the optimal environment for binding, the yeast peptides (-1 pg of peptide) were mixed with 2µ1 of a 10µg/µ1 bead stock and treated with one of the following conditions: 1. An equal volume of a 5% formic acid solution was added to acidify the mixture (nanodiamond conditions), 2. A pre-determined volume of 100% acetonitrile was added to achieve a specific final percentage solution, 3. A small volume of 5% formic acid in combination with a pre-determined volume of 100% acetonitrile was added to achieve a specific final percentage solution, 4. An equivalent volume of a 2.5M NaCl, 120% ammonium sulfate solution was added (SPRI conditions). The rinsing conditions in SP3 are the same as those discussed below. In conditions 2 and 3, a specific concentration of acetonitrile must be reached. To determine these levels, recovery of the peptide mixture across a range of acetonitrile concentrations was tested (20 to 95%). As readout of recovery peptides eluted off beads after treatment were directly run on the MS. The signal intensity and complexity between runs was compared to determine the optimum percentage of acetonitrile. These curves were completed both in the presence and absence of formic acid (data not shown). A final concentration of 95% of acetonitrile in neutral pH conditions was found to give the highest and most reproducible recovery.

### Example 7: SP3 protocol: Peptides.

Bead-peptide solutions were mixed to re-suspend the beads that had settled during the digestion procedure. To each tube, 100% acetonitrile was added to achieve a final concentration >95% (e.g. 5µl of protein digest, 195µ1 of 100% acetonitrile). Mixtures were incubated for 8 minutes at room temperature and following this, placed on a magnetic rack for a further 2 15 minutes. The supernatant was discarded, and the beads rinsed one time with 180µ1 of 100% acetonitrile. Rinsed beads were reconstituted to elute peptides in a volume of 4% DMSO amenable for MS analysis. Mixtures were pipette mixed and incubated for 5 minutes at room temperature. Tubes were placed on a magnetic rack and eluted peptides recovered. Prior to analysis with MS, peptides solutions were acidified with formic acid.

### Example 8: SP3 Dimethyl Labeling.

Working solutions of 1% formaldehyde (Thermo Scientific) and 155mM sodium cyanoborohydride (Sigma) were prepared in water. Peptide solutions derived from SP3 digests were typically contained in a total volume of 5µ1 of digestion buffer. To each tube, 1µ1 of 1% formaldehyde (light) was added. In addition, a further 1µ1 of 155mM sodium cyanoborohydride was added, and reactions pipette mixed. Labeling reactions were incubated for 1 hour at room temperature. Reactions were quenched through addition of luL of a mixture of 10mM lysine (Sigma) and 50mM ammonium bicarbonate (Sigma). Labeled peptides were treated directly with SP3 peptide clean-up prior to MS analysis.

### Example 9: SP3 TMT Labeling.

TMT labeling kits were obtained from Thermo Scientific. Each TMT label (0.8mg per vial) was reconstituted in 40µ1 of acetonitrile. Peptide solutions derived from SP3 digests were typically contained in a total volume of 5µ1 of digestion buffer. Labeling reactions were carried out through addition of 20µg of TMT label per 10µg of peptide in two volumetrically equal steps of 1µ1, 30 minutes apart. TMT labeling reactions were carried out on the magnetic rack at all times to avoid potential interactions between peptides and the beads due to the addition of acetonitrile. Reactions were quenched through addition of 1µ1 of a mixture of 10mM lysine (Sigma) and 50mM ammonium bicarbonate (Sigma). Labeled peptides were treated directly with SP3 peptide clean-up prior to MS analysis.

### Example 10: High-pH reversed phase fractionation.

High-pH reversed phase analysis was performed either on an Agilent 1200 HPLC system equipped with a variable wavelength detector (254nm) or StageTips. On the HPLC, fractionation was performed on an XBridge BEH C18 column (1 x 100mm, 3.5um, 130A, Waters). Elution was performed at a flow rate of 0.1mL per minute using a gradient of mobile phase A (20mM ammonium formate, pH 10) and B (acetonitrile), from 1% to 37.5% over 61 minutes. Fractions were collected every 2 minutes across the entire gradient length and concatenated into 10 final samples as discussed previously (Yang, F. & Shen, Y. *et al.,* 2012). Fractions were dried in a SpeedVac centrifuge and reconstituted in 0.1% formic acid prior to MS analysis. For StageTip fractionation, tips were rinsed 2 times with 100% methanol and 2 times with 20mM ammonium formate (pH 10) prior to sample loading. Fractions were eluted step-wise at acetonitrile concentrations of 11.1%, 14.5%, 17.4%, 20.8%, and 85% in 20mM ammonium formate (pH 10). Fractions 1 and 5 were combined prior to analysis. Each fraction was dried in a SpeedVac and reconstituted in 0.1% formic acid prior to MS analysis.

### Example 11: Mass spectrometry analysis.

For optimization of the SP3 protocol, samples were run on a high-capacity trap (HCT) Ultra Ion Trap MS (Bruker Daltonics). Full scan MS spectra were acquired with a mass range of 350 to 1500 m/z in profile mode. The maximum fill time was set to 200 milliseconds with a target value of 2e5. Fragment-MS spectra were acquired over the mass range of 100 to 2000 m/z with a maximum fill time of 30 milliseconds. All HCT data were processed manually offline using in-house scripts. For analysis using the qExactive MS (Thermo Scientific) with higher-energy collisional dissociation (HCD) fragmentation, samples were introduced using an UltiMate 3000 LC system (Dionex). Injected peptides were trapped on Acclaim PepMap C18 columns (75um x 20mm, 3um, 100A, Dionex). After trapping, gradient elution of peptides was performed on a C18 (Reprosil-Pur, Dr. Maisch, 3um particle size) column packed in-house in 100um internal diameter capillaries where one end has been pulled with a laser-puller to function as a nano-emitter. Elution was performed with a gradient of mobile phase A (99.9% water and 0.1% formic acid) to 25% B (99.9% acetonitrile and 0.1% formic acid) over 50 minutes, and to 40% B over 15 minutes, for a final length of 90 minutes. Alternatively, the gradient was ramped to 25% B over 100 minutes, and 40% B over 24 minutes, for a final length of 145 minutes. For TMT labeled samples, the gradient was adjusted to run to a concentration of 27% mobile phase B at the 100 minute step. Data acquisition on the qExactive MS was carried out using a data-dependent method. The top 12 precursors were selected for tandem-MS/MS (MS2) analysis after HCD fragmentation. Survey scans covering the mass range of 300 to 1600 were acquired at a resolution of 35,000 (at m/z 200), with a maximum fill time of 30 milliseconds, and an automatic gain control (AGC) target value of 1e6. MS2 scans were acquired at a resolution of 17,500 (at m/z 200), with a maximum fill time of 200 milliseconds, and an AGC target value of 5e4. An isolation window of 2.0 m/z with a fixed first mass of 110.0 m/z we applied in all experiments. HCD fragmentation was induced with a normalized collision energy (NCE) of 25 for unlabeled and dimethyl samples and 33 for TMT peptides. The underfill ratio was set at 40% to achieve an intensity threshold of 1e5. Dynamic exclusion was set to exclude the previously selected precursors for a total of 30 or 60 seconds, depending on gradient length. Charge state exclusion was set to ignore unassigned, 1, 5 to 8, and >8 charges. Isotope exclusion was enabled and peptide match was disabled. All data were acquired in profile mode. Experiments involving the analysis of limited amounts of material (HeLa and drosophila) were carried out on an Orbitrap Velos Pro MS system (Thermo Scientific) equipped with a nanoAcquity liquid chromatography system (Waters). Injected peptides were trapped on Symmetry C18 columns (180um x 20mm). After trapping, gradient elution of peptides was performed on a C18 (nanoAcquity BEH130 C18, 75um x 200mm, 1.7um) column. For single-shot samples where extended analysis was used, elution was performed with a gradient of mobile phase A (99.9% water and 0.1% formic acid) to 25% B (99.9% acetonitrile and 0.1% formic acid) over 190 minutes, and to 40% B over 40 minutes, for a final length of 265 minutes. For samples fractionated with high-pH reversed phase, 145 minute gradient runs were used, as discussed above. Data acquisition on the Orbitrap Velos Pro MS was carried out using a data-dependent method. The top 15 precursors were selected for MS2 analysis after collisional induced fragmentation (CID). Survey scans covering the mass range of 300 to 1500 were acquired at a resolution of 30,000 (at m/z 400) with a maximum fill time of 500 milliseconds, and an AGC target value of 1e6. MS2 scans were acquired at a resolution of 7,500 (at m/z 400) with a maximum fill time of 50 milliseconds and an AGC target value of le4 with an isolation window of 2.0 m/z. CID fragmentation was induced with an NCE of 40, an activation time of 10 milliseconds, and an activation Q of 0.250. Dynamic exclusion was set to exclude previously selected precursors for a total of 6 to 90 seconds depending on gradient length. Charge state exclusion was set to ignore unassigned, 1, and 4 and greater charges. MS1 data were acquired in profile mode, whereas MS2 were obtained in centroid format.

### Example 12: Proteomic Data Analysis

Data-dependent data were analysed using either PEAKS (ver. 7) software (Bioinformatic Solutions) or MaxQuant (ver. 1.4.1.2). With PEAKS, raw data were imported and refined using the following settings: Merge Scans - True, retention time window - 1 minute, precursor m/z tolerance - 5 ppm, Correct Precursor - True, mass only, Filter Scans - True, quality value greater than 0.65. Data were *de novo* sequenced using precursor and fragment mass tolerance windows of 20 ppm and 0.06 Daltons, respectively, for qExactive data. With the Orbitrap Velos Pro MS, tolerance windows of 20ppm and 0.6 Daltons were used for precursor and fragment mass. In both instances, enzyme specificity was set as trypsin, with carbamidomethylation as a fixed, and oxidation of methionine as a variable modification. The maximum allowed variable post-translational modification (PTM) per peptide was set to 3, with up to 5 candidates reported per spectrum. PEAKS database searches were performed using the same precursor and fragment mass tolerances as with the *de novo* sequencing. Enzyme specificity was set as trypsin with 2 missed cleavages allowed, and non-specific cleavage at one end of the peptide. The modifications were as with the *de novo* sequencing, with 5 variable PTMs per peptide. Identification and quantification was performed in MaxQuant (Cox, J. & Mann, M. MaxQuant enables high peptide identification rates, individualized p.p.b.-range mass accuracies and proteome-wide protein quantification, Nat. Biotechnol. 26, 1367-72, 2008). Raw data were searched using an initial tolerance of 20ppm and subsequently re-searched at 6ppm following recalibration. A fragment ion tolerance of 20ppm was used in all searches. Data were searched using the Andromeda engine built into MaxQuant (Cox, J. et al. Andromeda: a peptide search engine integrated into the MaxQuant environment. J. Proteome Res. 10, 1794-805, 2011). Carbamidomethylation of cysteine was specified as a fixed modification, with oxidation of methionine and acetylation of the protein N-terminus as variable. In searches with TMT and dimethyl labeled samples, variable modifications of the N-terminus and lysine were specified for masses of +28.0313 (dimethyl) and +229.1629 (TMT). In samples requiring quantification, re-quantify was enabled. Matching between runs was enabled in all searches with specified match and time windows of 2 and 20 minutes, respectively. The databases searched were complete proteomes including isoforms from UniProt (Human: 88933, Drosophila: 41509, Yeast: 6767 total sequences). All databases were appended with a list of common contaminants (cRAP, The GPM). Error rate was estimated using a concatenated target-decoy database strategy in PEAKS, and a reversed database in MaxQuant. Data were filtered to a maximum false discovery rate of 0.01 for proteins and peptides. Each protein was required to be matched by a minimum of 2 unique peptides. Resulting data sets were exported and analyzed with a combination of scripts built in Python and R designed in-house.

### References

1. Lamond, A. I. et al., Molecular & Cellular Proteomics, 11 (3) (2012).
2. Smith. L.M. et al., Nature Methods, 10 (3): 186-187 (2013).
3. Yates, J.R. et al., Annual review of biomedical engineering, 11:49-79 (2009).
4. Geier, T. et al., Molecular & Cellular Proteomics, 11 (3) (2012).
5. Nagaraj, N. et al., Molecular & Cellular Proteomics, 11(3) (2012).
6. Zhou, F. et al., Nature Communications, 4, (2013).
7. Wisniewski, J.R. et al., Nature methods 6 (5) 359-362 (2009).
8. Hengel, S.M. et al., Proteomics 12 (21) 3138-3142 (2012).
9. 5 9. Bereman, M.S., Proteomics 11 (14) 931-2935 (2011).
10. Hawkins, T.L. et al., Nucleic acids research 22 (21) 4543 (1994).
11. DeAngelis, M.M. et al., Nucleic acids research 23 (22) 4742-4743 (1995).
12. Wilkening, S. et al., BMC genomics 14(1) 90 (2013).
13. Chen, W.-H. et al., Analytical Chemistry, 78:4228, (2006).
14. Current Protocols in Protein Science, Supplement 7, uni 4.5, chapter 4.5.5 " Selective precipitation by salting out", Wiley publishing company, (1997).
15. Altelaar, A. et al., Current opinion in chemical biology 16 (1) 206-213, 2012.
16. Yang, F. et al., Expert Review of Proteomics 9 (2) 129-134, (2012).
17. Cox, J. et al., Nature Biotechnology 26, 1367-72, 2008).
18. Cox, J. et al. Journal of Proteome Res. 10, 1794-805, (2011).

## Claims

1. A method of reversibly binding polypeptides to a solid phase comprising a hydrophilic surface, wherein the solid phase consists of paramagnetic microbeads or microparticles and wherein the method is carried out in a single tube, comprising the step of:
contacting said solid phase with a solution containing said polypeptides and a dehydration solution and/or a precipitation solution, wherein
(i) the dehydration solution comprises a polar organic solvent selected from acetonitrile, acetone and alcohol and/or a hygroscopic polymer, said hygroscopic polymer being polyethylene glycol; and/or
(ii) the precipitation solution comprises ions of high ionic strength selected from the group consisting of ammonium, potassium, sodium, fluoride, sulphate, hydrogen phosphate, acetate, chloride, and combinations thereof.

2. The method of claim 1, wherein the solid phase consists of carboxylated paramagnetic microbeads and wherein the dehydration solution comprises acetonitrile.

3. The method of claim 1 or 2, wherein the dehydration solution or precipitation solution is added in an amount resulting in the reversible binding of at least 90% of the polypeptides in said solution.

4. The method of any of claims 1 to 3, wherein the solution containing said polypeptides is selected from a whole cell extract, a whole cell extract digest, proteins derived from tissue, recombinant purified proteins, purified proteins, a protein digest, a purified protein digest, and a peptide library.

5. The method of any of claims 1 to 4, wherein the solid phase comprising reversibly bound polypeptides is separated from the solution.

6. The method of any one of claims 1 to 5, wherein the solid phase is washed with a washing solution comprising a polar organic solvent or a salt comprising at least one ion or a chaotrope comprising at least one ion that increases hydrophobic interaction of polypeptides, preferably in a concentration range from 1 M - 8 M.

7. The method of any of claims 1 to 6, comprising the further step of adding a protease.

8. The method of any of claims 1 to 7, wherein the polypeptides are released from the solid phase with an aqueous elution solution.

9. The method of claim 7 or 8, comprising the further step of submitting the aqueous solution to mass spectrometry.

10. A kit comprising
a. a solid phase comprising a hydrophilic surface, wherein the solid phase consists of paramagnetic microbeads or microparticles; and
b. a dehydration solution comprising a polar organic solvent, wherein said polar organic solvent is selected from acetonitrile, acetone and alcohol; and
c. a protease.

11. The kit of claim 10, wherein said kit further comprises one or both of the following:
a. a hygroscopic polymer which is polyethylene glycol; and
b. a precipitation solution that comprises ions of high ionic strength selected from ammonium, potassium, sodium, fluoride, sulphate, hydrogen phosphate, acetate, chloride, or combinations thereof.

12. Use of
a. a solid phase, and
b.
(i) a dehydration solution; and/or
(ii) a precipitation solution, wherein
the solid phase comprises a hydrophilic surface, wherein the solid phase consists of paramagnetic microbeads or microparticles comprising the hydrophilic surface;
the dehydration solution comprises a polar organic solvent selected from acetonitrile, acetone and alcohol and/or a hygroscopic polymer, said hygroscopic polymer being polyethylene glycol; and/or
the precipitation solution comprises ions of high ionic strength selected from the group consisting of ammonium, potassium, sodium, fluoride, sulphate, hydrogen phosphate, acetate, chloride, and combinations thereof,
for the analysis, identification, characterisation, quantification, purification, concentration and/or separation of polypeptides carried out in a single tube.
